# EUROPEAN PATENT SPECIFICATION

(11) **EP 4 440 448 B1**
(45) Date of publication and mention of the grant of the patent: **08.04.2026**
(21) Application number: 22847288.2
(22) Date of filing: 29.11.2022
(51) Int. Cl.: A61B 17/04, A61F 2/24

(54) **SURGICAL PAD REINFORCEMENT**
VERSTÄRKUNG FÜR CHIRURGISCHES KISSEN
RENFORT DE TAMPON CHIRURGICAL

(30) Priority: 03.12.2021 US 202163264919 P
(43) Date of publication of application: 09.10.2024
(73) Proprietor: Edwards Lifesciences Corporation, Irvine, CA 92614 (US)
(72) Inventor: CORTEZ, Felino V., Irvine, CA 92614 (US); COURNANE, Stephen, Irvine, CA 92614 (US)
(74) Representative: Eisenführ Speiser
(86) International application number: PCT/US2022/051141
(87) International publication number: WO 2023/101921

(56) References cited:
- EP-B1- 1 480 565
- US-A1- 2014 180 436
- US-A1- 2015 119 928
- US-A1- 2019 328 374
- US-B1- 7 930 016

## Description

### TECHNICAL FIELD OF THE INVENTION

The present disclosure generally relates to the field of reinforcement of surgical pads used in proximity to tissue openings.

### BACKGROUND

Surgical tethers can be anchored to a target tissue as a part of medical procedures for treating various health conditions. Anchoring surgical tethers to the heart wall can be a part of heart valve repair procedures to address a number of heart valve abnormalities. The surgical tethers can be anchored to the heart wall so as to maintain a desired tension in the surgical tethers.

EP 1480 565 B1 describes an occlusion device comprising first and second disk members, each of the first and second disk members having a self-expanding, non-metallic structure including a plurality of integral rib members which are flexible so as to be compressible within a catheter and are biased towards an open position to expand the disk members when removed from the catheter, and a central rim which is collapsible and by means of which the first and second disk members are connected together, and wherein the occlusion device includes a sinuous structure around the central rim.

US 2019/0328374 A1 relates to an implantable closure device comprising a self-expanding metal frame comprising a central portion defining a lumen and a plurality of first anchoring arms and a plurality of second anchoring arms angularly spaced around the central portion. The central portion comprises a sinusoidal-shaped ring defining a plurality of apices. The first anchoring arms are connected to locations inside of the apices on a first side of the central portion and the second anchoring arms are connected to locations inside of the apices on a second side of the central portion. The first anchoring arms are configured to bear against a first surface of a septal wall. The second anchoring arms are configured to bear against a second surface of the septal wall.

US 7,930,016 B1 describes a tissue closure system, comprising a deployment catheter defining at least one lumen therethrough, a hood comprising a non-inflatable membrane forming a fluid barrier projecting distally from the deployment catheter and adapted to self-expand into an expanded deployed configuration defining an open area therein, wherein the open area is in fluid communication with the at least one lumen and with an environment external to the hood through an opening defined by the hood, a visualization element disposed within or adjacent to the open area of the hood for visualizing tissue adjacent to the open area, and a tissue approximation assembly deployable from within the hood and configured to secure a tissue opening.

US 2015/0119928 A1 relates to a vasculature closure device that includes an expandable support frame deployable within a vessel and a sealing membrane at least partially supported by the expandable support frame. Upon expanding the support frame, the vasculature closure device is configured to intraluminally secure the sealing membrane against a puncture site existing in a vessel wall.

US 2014/0180436 A1 relates to a prosthesis that includes a distal patch that is advanced from the inguinal canal through and beyond the hernia defect in a collapsed configuration. The patch is thereafter opened or expanded so that it can be deployed over the defect. Once expanded, the patch is retracted back and positioned against the distal or posterior surface of the abdominal wall to cover the defect. For repairing an indirect inguinal hernia, the patch is advanced through and deployed over the inguinal ring.

### SUMMARY

The present invention pertains to a reinforced surgical pad device as defined in claim 1. Some advantageous aspects are defined in the dependent claims 2-15.

In some examples, described herein are systems and methods relating to reinforced surgical pad devices comprising a reinforcement member and a surgical pad configured to be associated with the reinforcement member, including a central portion of the reinforcement member. According to the claimed invention, the surgical pad is configured to be coupled to the reinforcement member, including a central portion of the reinforcement member. The reinforcement member and surgical pad are configured to be positioned at least partially over an externally oriented surface of a target tissue and an opening formed in the target tissue. One or more tethers extending from the opening in the target tissue can be coupled to the surgical pad and/or reinforcement member. The reinforcement member can provide mechanical reinforcement and/or support to reduce or eliminate deformation of the surgical pad. In some instances, the reinforcement member comprises one or more portions configured to extend laterally beyond an outer edge of the surgical pad, to thereby provide a larger area over which forces exerted upon the surgical pad by the tethers coupled thereto can be distributed. Distribution of forces exerted upon the surgical pad over a wider area can thereby reduce or prevent warping of the surgical pad due to concentrated force exerted thereupon by the one or more tethers.

Methods and structures disclosed herein for treating a patient also encompass analogous methods and structures performed on or placed on a simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (*e.g.,* thorax), a system (*e.g.,* cardiovascular system), an organ (*e.g.,* heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loud speakers, headphones, pressure transducers, temperature transducers, or using any combination of suitable technologies.

For purposes of summarizing the disclosure, certain aspects, advantages and novel features have been described herein. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the disclosure. In addition, various features of different disclosed examples can be combined to form additional examples, which are part of this disclosure. Throughout the drawings, reference numbers may be reused to indicate correspondence between reference elements. However, it should be understood that the use of similar reference numbers in connection with multiple drawings does not necessarily imply similarity between respective examples associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some examples or configurations.
Figure 1 is a cross-sectional view of a human heart and two tethers deployed to couple a mitral valve leaflet and a mitral valve annulus to a left ventricular wall.
Figure 2A is a top-down view, and Figure 2B is a perspective view, of an example of a reinforcement member.
Figure 3A is a top-down view, and Figure 3B is a cross-sectional view, of an example of a reinforced surgical pad device comprising the reinforcement member of Figure 2.
Figures 4A is a top-down view, and Figure 4B is a perspective view, of an example of a reinforcement member.
Figure 5A is a top-down view, and Figure 5B is a cross-sectional view, of an example of a reinforced surgical pad device comprising the reinforcement member of Figure 4.
Figure 6A is a top-down view, and Figure 6B is a perspective view, of an example of a reinforced surgical pad device.
Figures 7A and 7B are top-down views, and Figure 7C is a cross-sectional view, of a plurality of tethers coupled to the reinforced surgical pad device of Figure 6.
Figures 8A is a top-down view, and Figure 8B is a perspective view, of an example of a reinforcement member.
Figures 9A and 9B are top-down views, and Figure 9C is a cross-sectional view, of a plurality of tethers coupled to the reinforced surgical pad device of Figure 8.
Figure 10A is a top-down view, and Figure 10B is a cross-sectional view, of an example of a reinforced surgical pad device and a plurality of tethers coupled thereto.
Figures 11A is a top-down view, and Figure 11B is a perspective view, of an example of a reinforcement member.
Figures 12A and 12B are top-down views, and Figure 12C is a cross-sectional view, of a plurality of tethers coupled to the reinforced surgical pad device of Figure 11.
Figure 13A is a top-down view, and Figure 13B is a cross-sectional view, of an example of a reinforced surgical pad device and a plurality of tethers coupled thereto.
Figure 14A is a perspective view, and Figure 14B is a top-down view, of an example of a reinforced surgical pad device comprising a reinforcement member and a surgical pad having a plurality of pre-formed openings extending therethrough.
Figure 15 is a process flow diagram of an example of a process to deploy a reinforced surgical pad device.

### DETAILED DESCRIPTION

The headings provided herein are for convenience only and do not necessarily affect the scope or meaning of the claims.

Although certain preferred examples are disclosed below, the scope of the claims is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

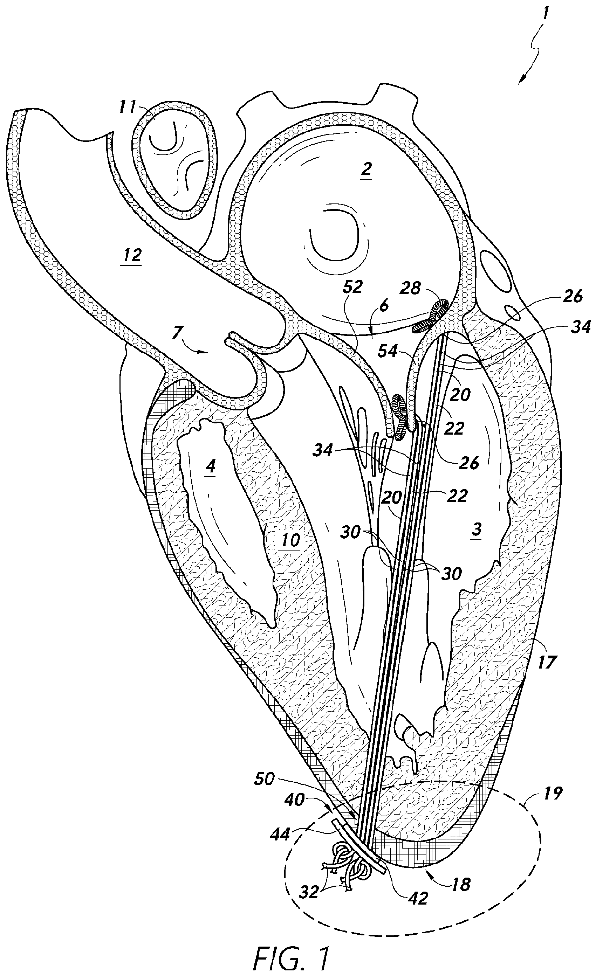

Figure 1 shows a cross-sectional view of a human heart 1. The cross section is taken along a sagittal plane. The heart 1 includes four chambers, namely the left atrium 2, the left ventricle 3, the right ventricle 4, and the right atrium. A wall of muscle, referred to as the septum 10, separates the left atrium 2 and right atrium, and the left ventricle 3 and right ventricle 4. Blood flow through the heart 1 is at least partially controlled by four valves, the mitral valve 6, aortic valve 7, tricuspid valve, and pulmonary valve. The mitral valve 6 separates the left atrium 2 and the left ventricle 3 and controls blood flow therebetween. The aortic valve 7 separates and controls blood flow between the left ventricle 3 and the aorta 12. The tricuspid valve separates the right atrium and the right ventricle 4 and controls blood flow therebetween. The pulmonary valve separates the right ventricle 4 and the pulmonary trunk or artery 11, controlling blood flow therebetween.

In a healthy heart, the heart valves can properly open and close in response to a pressure gradient present during various stages of the cardiac cycle (*e.g.,* relaxation and contraction) to at least partially control the flow of blood to a respective region of the heart and/or to blood vessels. Deoxygenated blood arriving from the rest of the body generally flows into the right side of the heart 1 for transport to the lungs, and oxygenated blood from the lungs generally flows into the left side of the heart 1 for transport to the rest of the body. During ventricular diastole, deoxygenated blood arrive in the right atrium from the inferior vena cava and superior vena cava to flow into the right ventricle 4, and oxygenated blood arrive in the left atrium 2 from the pulmonary veins to flow into the left ventricle 3. During ventricular systole, deoxygenated blood from the right ventricle 4 can flow into the pulmonary trunk 11 for transport to the lungs (*e.g.,* via the left and right pulmonary arteries), and oxygenated blood can flow from the left ventricle 3 to the aorta 12 for transport to the rest of the body.

A number of conditions can contribute to a higher-than-normal pressure in the left atrium 2. Dysfunction of the mitral valve 6 can contribute to elevated left atrial pressure. Conditions such as mitral valve regurgitation and/or stenosis may result in difficulty in pumping blood from the left atrium 2 to the left ventricle 3, contributing to elevated pressure in the left atrium 2. Valve stenosis can cause a valve to become narrowed or obstructed. Mitral valve stenosis can restrict blood flow from the left atrium 2 to the left ventricle 3. Valve regurgitation occurs when a valve does not close properly. For example, regurgitation can occur due to improper coaptation of the valve leaflets, such as due to prolapse of one or more of the valve leaflets. Mitral valve regurgitation can result in blood flow leakage back into the left atrium 2 from the left ventricle 3 when the left ventricle 3 contracts. Restricted flow of blood from the left atrium 2 into the left ventricle 3, and blood flow leakage from the left ventricle 3 back into the left atrium 2 can both contribute to elevated atrial pressure. Dysfunction in the left ventricle 3 can also contribute to elevated left atrial pressure. Elevated left atrial pressure may lead to left atrial enlargement, producing symptoms such as shortness of breath during exertion, fatigue, chest pain, fainting, abnormal heartbeat, and swelling of the legs and feet.

One or more tethers can be deployed to couple a heart valve leaflet and/or heart valve annulus to the heart wall 17 to improve coaptation of the heart valve leaflets. For example, one or more tethers can be deployed to couple both a mitral valve leaflet and a portion of the mitral valve annulus to the left ventricular wall. The tethers are typically advanced from the left ventricle 3 through the heart wall 17 adjacent to the left ventricle 3 and secured to a surgical pad positioned over an externally oriented surface of the heart wall 17 adjacent to the left ventricle 3, such as the left ventricular heart wall. Force exerted upon the location on the heart wall 17 due to tethering thereto of the leaflet and annulus can lead to deformation of that portion of the heart wall 17, contributing to indenting of the heart wall 17. Highly tensioned tethers coupled to the ventricular wall can cause that area of the ventricle to cave in, thereby leading to failure of the repair to restore mitral valve function. For example, force exerted upon the heart wall 17 due to tethering of the annulus can result in pulling inward into the ventricle of the heart wall 17. Deformation of the heart wall 17 can reduce tension in the tethers, thereby preventing desired force from being exerted upon the leaflet and/or annulus to pull in the leaflet and/or annulus for improving leaflet coaptation.

Described herein are systems and methods relating to one or more reinforced surgical pad devices. The reinforced surgical pad devices comprise a reinforcement member and a surgical pad configured to be associated with the reinforcement member. The reinforcement member and surgical pad are configured to be positioned over an opening in a target tissue such that one or more tethers extending from the opening in the target tissue can be secured to the surgical pad and/or reinforcement member. The reinforcement member comprises one or more portions configured to extend laterally beyond an outer edge of the surgical pad. The reinforcement member can be configured to provide a larger area over which forces exerted upon the surgical pad by the tethers coupled thereto can be distributed. Distribution of the forces across a larger area can reduce or eliminate concentration of force upon the target tissue. Concentration of force upon the target tissue can lead to indentation of the target tissue. As described herein, one or more tethers coupled to a heart valve leaflet and/or heart valve annulus can be anchored to a location on the ventricular heart wall. The larger footprint provided by the reinforcement member can facilitate spreading of the forces exerted upon the anchor site across a larger area of the heart wall to prevent or reduce indentation of the heart wall inward toward the ventricle.

The reinforcement member comprises a central portion and a circumferential portion around the central portion, where the central portion is configured to be associated with the surgical pad. According to the claimed invention, the central portion of the reinforcement member defines an opening extending therethrough. The surgical pad is configured to be associated with the opening in the central portion. In some instances, the surgical pad can have a same or smaller width and/or length as those of the opening of the reinforcement member such that the surgical pad can be positioned within the opening of the reinforcement member. For example, the surgical pad can be positioned over the opening in the target tissue and the reinforcement member can be positioned over the target tissue such that an inner edge of the central portion defining the opening of the reinforcement member surrounds the opening in the target tissue.

In an example which is not according to the claimed invention, the surgical pad can have a longer width and/or length than the opening in the reinforcement member such that a portion of the surgical pad can be associated with a surface of the reinforcement member configured to be oriented toward the target tissue. For example, the surgical pad and the reinforcement member can be positioned over the target tissue such that a portion of the surgical pad is between the target tissue and the reinforcement member.

In some instances, the circumferential portion of the reinforcement member can comprise a plurality of elongate portions. The plurality of elongate portions can extend radially from the central portion. In some instances, the plurality of radially arranged elongate portions can facilitate desired distribution of force across the target tissue. In some instances, the circumferential portion can comprise an outer circumferential edge portion such that the plurality of elongate portions couples the central portion and the outer circumferential edge portion. For example, the reinforcement member can comprise a plurality of elongate portions extending radially from the central portion to the outer circumferential edge portion. In some instances, the outer circumferential edge portion can be configured to provide additional structural stability for the reinforcement member. In some instances, the reinforcement member can have a hub-and-spoke configuration. In some instances, the reinforcement member can have a hub-and-spoke wheel configuration.

Heart valve repair and/or replacement procedures can be performed to improve or restore valve function. Referring again to Figure 1, respective tethers 20 are shown coupling a leaflet of the mitral valve 6 and an annulus of the mitral valve 6 to the heart wall 17. In some instances, more than one tether can be deployed to each of a leaflet, including posterior leaflet, such as a mid-segment of the posterior leaflet, and a portion of the annulus of the mitral valve 6, including a posterior leaflet annulus. The posterior leaflet annulus can comprise a portion of the annulus of the mitral valve 6 adjacent to the posterior leaflet. An opening 50 can be formed through the heart wall 17 to access a heart valve. For example, mitral valve repair procedures can be performed to alleviate mitral valve dysfunction, including mitral valve prolapse. In some cases, a transapical approach can be used to gain access into the heart 1. For example, mitral valve repair procedures can include accessing the mitral valve 6 from within the left ventricle 3, where entry into the left ventricle 3 can be achieved through the left ventricular wall in the apical region 19 of the heart 1. The heart wall 17 can be punctured in the apical region 19, such as in the apical region 19 on the left ventricular wall, to form the opening 50 so as to allow delivery of medical devices and/or therapy to the mitral valve 6.

The apical region 19 is schematically shown in Figure 1 as the area within the dashed circle. As used herein, the "apical region" can include the inferior tip of the heart 1. The inferior tip is referred to herein as the apex 18 of the heart 1 and is generally located on the midclavicular line, in the fifth intercostal space. The apex 18 can be considered part of the greater apical region 19. Generally, the apical region 19 of the heart is a bottom region of the heart that is within the left or right ventricular region but is distal to the mitral 6 and tricuspid 8 valves and toward the tip of the heart 1. More specifically, the apical region 19 may be considered to comprise a bottom portion of the heart 1 that is within about 20 centimeters (cm) to the right or to the left of the median axis of the heart 1.

The tethers 20 in Figure 1 are shown as tethering the leaflet and annulus to the heart wall 17. For example, the tethers 20 coupled to the posterior leaflet and the posterior leaflet annulus can be tensioned anteriorly and downward into the left ventricle 3, coupling the leaflet and annulus to a left ventricular portion of the heart wall 17. Coupling the leaflet and annulus to the heart wall 17 can facilitate reshaping of the mitral valve 6, such as to reduce or eliminate leaflet prolapse and/or reduce a size of the valve orifice. The tethers 20 can serve to improve coaptation of the mitral valve leaflets. These tethers can be made from a variety of materials. One or more of these tethers can comprise for example expanded polytetrafluoroethylene (ePTFE). For example, each of the tethers 20 can be an ePTFE suture.

Each tether 20 can comprise a respective elongate portion 22 comprising a proximal portion 24 and a distal portion 26. The proximal portion 24 of a tether 20 can be configured to be coupled to the heart wall 17. The distal portion 26 of a tether 20 can be coupled to the leaflet or annulus of the mitral valve 6. In some examples, the distal portion 26 can be associated with a respective anchor 28 to facilitate securing the tether 20 to the leaflet or the annulus. An anchor 28 can be positioned at least partially over an upper surface of the leaflet or at least partially over an upper surface of the annulus. For example, as shown in Figure 1, a respective anchor 28 can be positioned over each of an atrial facing surface of the posterior mitral valve leaflet and an atrial facing surface of the posterior mitral valve annulus. In some cases, an anchor 28 can comprise a suture knot. In some cases, the suture knot can be formed using the same or similar material as the tether 20. In some cases, the suture knot can be integral with the tether 20. The tether 20 can be a unitary piece comprising the anchor 28 and the elongate portion 22. For example, a portion of a suture can form the anchor 28, such as forming the suture knot, and another portion of the suture can form the elongate portion 22.

The proximal portion 24 of a tether 20 can be anchored to a portion of the heart wall 17, including a portion of the heart wall 17 at or proximate to the apex 18 of the heart 1, such as in the apical region 19. In some examples, a portion of an elongate portion 22 can extend at least partially through the heart wall 17. Figure 1 shows the tethers 20 each extending through an entire thickness of the heart wall 17. The proximal portion 24 of each tether 20 can be anchored at a position adjacent to an externally facing surface of the pericardium, epicardium, or myocardium. In some cases, each elongate portion 22 can comprise a pair of tether tails 30. For example, each tether tail 30 can comprise a proximal portion 32 and a distal portion 34. Each of the distal portions 34 of the tether tails 30 can be associated with a corresponding anchor 28, such as a suture knot. The proximal portions 32 can be anchored to the heart wall 17. In some instances, the anchor 28 can be integrated with the tether tails 30 to form a unitary piece. In some instances, the anchor 28 and suture tails 30 can be formed from respective portions of a suture. In some instances, the anchor 28 can comprise a bulky-knot anchor. For example, the bulky-knot anchor can be formed using the same suture that forms the tether tails 30.

In some instances, the proximal portion 24 of a tether 20 can be coupled to a reinforced surgical pad device 40 to facilitate anchoring the leaflet and annulus of the mitral valve 6 to the heart wall 17. The tethers 20 can couple the mitral valve leaflet and annulus to the reinforced surgical pad device 40, extending from the leaflet and annulus through the left ventricle 3 and opening 50 in the heart wall 17 to the reinforced surgical pad device 40. The reinforced surgical pad device 40 can be positioned over the opening 50 formed in the heart wall 17. In some cases, the reinforced surgical pad device 40 can be positioned over and/or adjacent to the pericardium, epicardium, or myocardium. For example, the proximal portions 24 of the tethers 20 can be coupled to a reinforced surgical pad device 40 on and in contact with the pericardium, epicardium, or myocardium to facilitate anchoring the proximal portions 24 to the target location within the apical region 19 and maintain desired tension in the tethers 20. The proximal portions 24 of the tethers 20 can be extended through the opening 50 in the heart wall 17, and through the reinforced surgical pad device 40.

The reinforced surgical pad device 40 can comprise a reinforcement member 44 and a surgical pad 42. The surgical pad 42 can be positioned over and in contact with the target tissue, for example over the opening 50 in the heart wall 17. The reinforcement member 44 can be positioned over the surgical pad 42 such that the surgical pad 42 is between the reinforcement member 44 and the target tissue. As described herein, in some instances, a reinforcement surgical pad device according to the claimed invention comprises a surgical pad disposed within an opening of a reinforcement member, such that the surgical pad can be positioned over the opening 50 in heart wall 17 and in contact with the heart wall 17 while the reinforcement member can be positioned over and in contact with the heart wall 17 and surrounding the opening 50. Although Figure 1 shows tethers 20 for anchoring the heart valve leaflet to the heart wall 17, it will be understood that the tethers 20 can be used to provide tethering for one or more leaflets and/or annulus of a valve other than the mitral valve, such as the tricuspid valve.

A reinforcement member can comprise any number of materials configured to provide desired mechanical support at the anchor point, while reducing or preventing damage and/or irritation to the target tissue. The reinforcement member can comprise a rigid and/or semi-rigid biocompatible material. In some instances, the reinforcement member can comprise a synthetic polymer, including for example, one or more of polyethylene, polypropylene, polystyrene, neoprene, and nylon. The reinforcement member can be a polymeric reinforcement member. In some instances, the reinforcement member can comprise a metal, including for example, one or more of aluminum, titanium, stainless steel, and nitinol. For example, the reinforcement member can be a metal or metal alloy reinforcement member. Alternatively, or in combination, the reinforcement member can comprise tissue and/or textile.

A surgical pad can comprise any number of materials. The surgical pad can be configured to comprise sufficient flexibility and mechanical strength to facilitate anchoring one or more tethers to the target site on a ventricular heart wall, while reducing or preventing damage to the target tissue. In some instances, a surgical pad can comprise a flexible material, such as polyurethane (*e.g.,* polyurethane foam), felt, and/or polytetrafluoroethylene (PTFE, *e.g.,* Teflon^{®} PTFE). In some instances, the surgical pad can comprise a pledget.

A surgical pad can have any number of different shapes, sizes and/or configurations. In some instances, the surgical pad can comprise a circular shape. In some instances, the surgical pad can have a rectangular shape. In some instances, the surgical pad can comprise a plurality of pre-formed openings extending therethrough, each opening being configured to receive a corresponding tether (*e.g.,* pair of tether tails) to facilitate threading through of the tether and securing of the tether to the surgical pad.

A reinforced surgical pad device comprising a reinforcement member and a surgical pad configured to be associated with the reinforcement member can be provided as an integrate device. The reinforcement member can be coupled to the surgical pad using any number techniques. The reinforcement member and the surgical pad can be bonded (*e.g.,* using adhesive), molded, fused (*e.g.,* using heat and/or pressure, such as welding, including radiofrequency (RF) welding), and/or mechanically fastened (*e.g.,* sewn), together. In some instances, the reinforcement member and the surgical pad may not be coupled to one another.

The methods, operations, steps, *etc.* described herein can be performed on a living animal or on a non-living cadaver, cadaver heart, simulator (*e.g.,* with the body parts, tissue, *etc.* being simulated), *etc.* For example, methods for treating a patient include methods for simulating the treatment on a simulated patient or anthropogenic ghost, which can include any combination of physical and virtual elements. Examples of physical elements include human or animal cadavers; any portions thereof, including organ systems, whole organs, or tissue; and manufactured elements, which can simulate the appearance, texture, resistance, or other characteristic. Virtual elements can include visual elements provided on a screen, or projected on a surface or volume, including virtual reality and augmented reality implementations. Virtual elements can also simulate other sensory stimuli, including sound, feel, and/or odor.

Any of the various systems, devices, apparatuses, *etc.* in this disclosure can be sterilized (*e.g.,* with heat, radiation, ethylene oxide, hydrogen peroxide, *etc*.) to ensure they are safe for use with patients, and the methods herein can comprise sterilization of the associated system, device, apparatus, *etc.* (*e.g.,* with heat, radiation, ethylene oxide, hydrogen peroxide, *etc*.).

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

Although the target tissue is described primarily herein as comprising heart ventricular wall tissue, it will be understood that the target tissue can comprise any number of different types of tissues in the heart and/or in other organs.

In some examples, systems, devices, and methods for deploying one or more tethers, as described herein, can comprise one or more features as described in PCT Patent Application No. PCT/US2021/023392, filed March 22, 2021, and entitled "Controlled Suture Tensioning,".

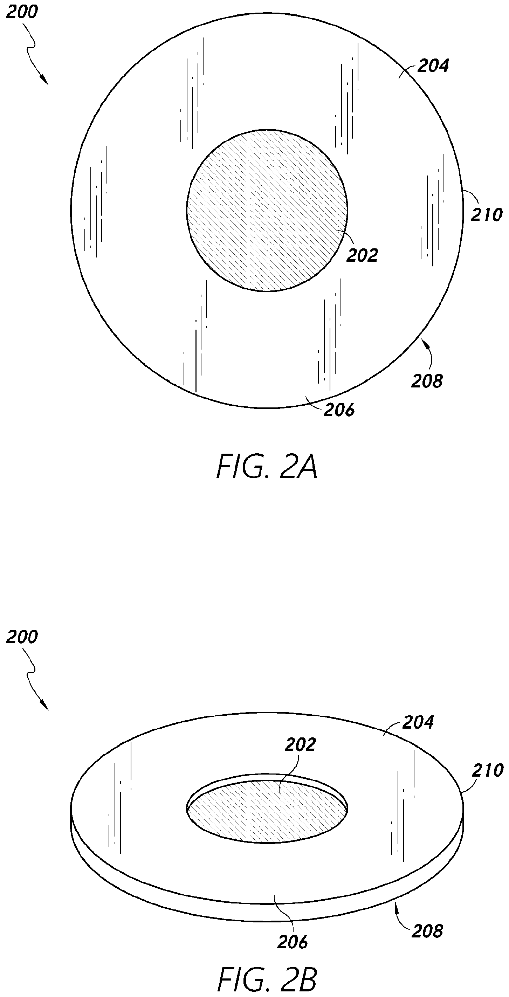

Figure 2A shows a top-down view, and Figure 2B shows a perspective view, of an example of a reinforcement member 200 configured to be associated with a surgical pad, wherein this example does not comprise all features of the claimed invention. The reinforcement member 200 can comprise a central portion 202 and a circumferential portion 204 around the central portion 202. The reinforcement member 200 can be configured to be positioned over a target tissue, including an opening formed in the target tissue, such that a first surface 206 of the reinforcement member 200 can be oriented toward the target tissue and a second surface 208 of the reinforcement member 200 can be oriented away from the target tissue. A surgical pad can be associated with the first surface 206 of the reinforcement member 200. For example, the surgical pad and the reinforcement member 200 can be positioned over the target tissue such that the surgical pad is between the target tissue and the reinforcement member 200. As described in further detail herein, one or more tethers extending from the opening in the target tissue can be coupled to the surgical pad. The reinforcement member 200 can be configured to provide structural support for the surgical pad so as to prevent or reduce deformation of the surgical pad due to forces exerted thereupon due to coupling of one or more tethers thereto.

The reinforcement member 200 can comprise a shape having one or more curved edges, such as a circular or substantially circular shape. The outer edge 210 of the reinforcement member 200 can comprise a circular or substantially circular shape. It will be understood that a reinforcement member can have other shapes. For example, a reinforcement member can comprise an outer edge having an oval shape and/or a polygonal shape, including a rectangular shape.

The central portion 202 can comprise any number of different shapes, including a circular shape. The central portion 202 may or may not have the same shape as the outer edge 210 of the reinforcement member 200. In some instances, both the central portion 202 and the circumferential portion 204 can have a circular shape.

In some instances, the central portion 202 can comprise one or more characteristics different from that of the circumferential portion 204. Referring to Figure 2B, in some instances, the central portion 202 can comprise a thickness thinner than that of the circumferential portion 204. A thickness of the reinforcement member 200 can be a dimension extending between opposing portions of the first and second surfaces 206, 208 of the reinforcement member 200. For example, a thickness extending between opposing portions of the first and second surfaces 206, 208 can be shorter in the central portion 202 than that in the circumferential portion 204. For example, the central portion 202 can define a recess on the first surface 206 of the reinforcement member 202. In some instances, the central portion 202 can comprise one or more other characteristics that are different from that of the circumferential portion 204, including for example, a different material. In some instances, a thinner thickness and/or a material of the central portion 202 can be selected so as to facilitate advancement therethrough of one or more tethers. A thicker thickness and/or material of the circumferential portion 204 can be selected so as to provide desired structural support for the surgical pad associated therewith. Alternatively, the central portion 202 can be the same as the circumferential portion 204. For example, the reinforcement member 200 can have a uniform or substantially uniform thickness and/or material composition.

In some instances, the one or more tethers can be coupled to both the surgical pad and the reinforcement member 200. The one or more tethers can be secured to the surgical pad and/or reinforcement member 200 using any number of techniques. As described in further detail herein, securing the one or more tethers to the surgical pad can comprise forming one or more knots using the one or more tethers. In some instances, the reinforcement member 200 can comprise one or more tether securement features (not shown) associated therewith, each tether securement feature being configured to allow a respective tether to be secured thereto. In some instances, the one or more tether securement features can be associated with the circumferential portion 204 of the reinforcement member 200. The one or more tether securement features can be associated with the second surface 208, such as the second surface 208 of the circumferential portion 204. The one or more tether securement features can comprise any number of configurations configured to facilitate securing thereto of a respective tether. As described herein, a tether securement feature can have a "T" shape. It will be understood that a tether securement feature can have other configurations, including for example, a hoop.

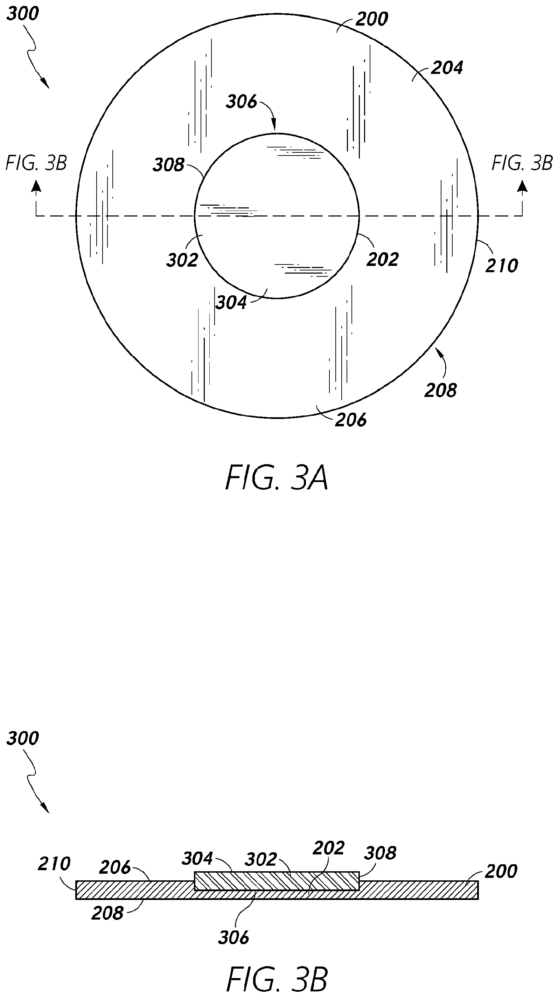

Figure 3A shows a top-down view, and Figure 3B shows a perspective view, of an example of a reinforced surgical pad device 300 comprising the reinforcement member 200 described with reference to Figure 2 and a surgical pad 302, wherein this example does not comprise all features of the claimed invention. As described herein, the surgical pad 302 can be associated with the central portion 202 of the reinforcement member 200, including the first surface 206 of the central portion 202. For example, the surgical pad 302 can be configured to be disposed over and/or be in contact with the first surface 206 of the reinforcement member 200. A first surface 304 of the surgical pad 302 can be configured to be oriented toward the target tissue and a second surface 306 of the surgical pad 302 can be configured to be oriented toward the reinforcement member 200, including the central portion 202 of the reinforcement member 200. The surgical pad 302 can be configured to be aligned with the central portion 202 of the reinforcement member 200. In some instances, the surgical pad 302 can be received at least partially a recess defined by the central portion 202. At least a portion of the reinforcement member 200 can extend laterally beyond an outer edge 308 of the surgical pad 302. For example, the circumferential portion 204 can comprise at least a portion configured to extend and/or be disposed laterally beyond the outer edge 308 of the surgical pad 302. While the surgical pad 302 is disposed over the central portion 202 of the reinforcement member 200, the outer edge 210 of the reinforcement member 200 can be laterally displaced from an outer edge 308 of the surgical pad 302. A lateral dimension, such as width, including a diameter, of the reinforcement member 200 can be longer than that of the surgical pad 302. Extension of the reinforcement member 200 laterally beyond the outer edge 308 of the surgical pad 302 can provide a larger footprint. The larger footprint can facilitate distribution of forces exerted upon the surgical pad 302 over a wider area, thereby reducing or preventing warping of the surgical pad 302 due to concentrated force exerted thereupon by the one or more tethers coupled thereto.

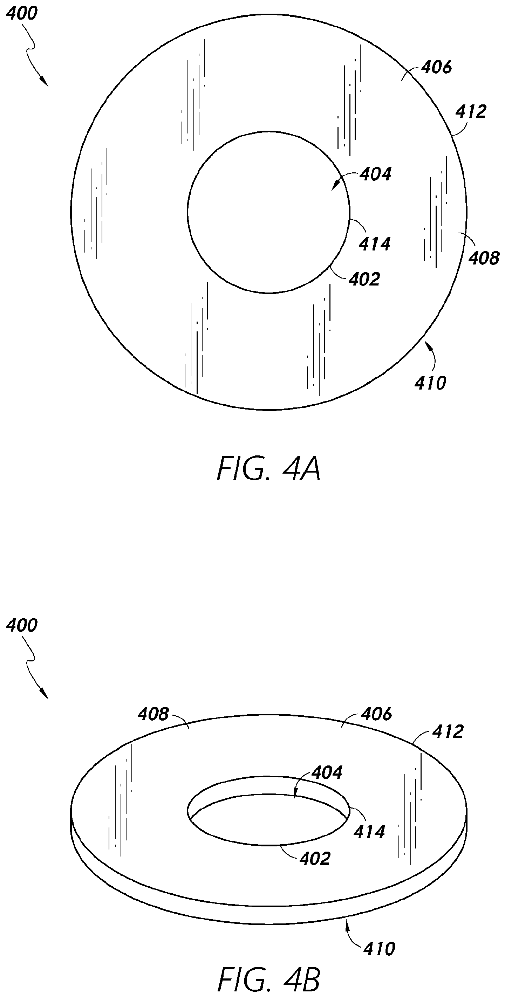

Figure 4A shows a top-down view, and Figure 4B shows a perspective view, of an example of a reinforcement member 400 comprising a central portion 402 defining an opening 404 extending therethrough. The reinforcement member 400 can comprise a circumferential portion 406 around the central portion 402. The central portion 402 can be configured to be associated with a surgical pad. In some instances, the surgical pad can be associated with the opening 404. The opening 404 can comprise any number of different shapes, including a circular shape. For example, an inner edge 414 defined by the central portion 402 can have a circle shape. In some instances, an opening of a reinforcement member can have a polygonal shape, such as a rectangular shape. As described in further detail herein, the surgical pad can be disposed within the opening 404.

The reinforcement member 400 can comprise one or more other features of the reinforcement member 200 described with reference to Figures 2 and 3. For example, the reinforcement member 400 can be configured to be positioned over a target tissue such that a first surface 408 can be configured to be oriented toward the target tissue and a second surface 410 can be configured be oriented away from the target tissue. An outer edge 412 of the reinforcement member 400 can comprise a shape having one or more curved edges, such as a circular or substantially circular shape. It will be understood that an outer edge of a reinforcement member can have other shapes. For example, a reinforcement member can comprise an outer edge having an oval shape and/or a polygonal shape, including a rectangular shape. The opening 404 defined by the inner edge 414 can have the same or different shape as that defined by the outer edge 412. In some instances, both inner edge 414 and the outer edge 412 can have a circular shape. For example, the reinforcement member 400 can comprise an annular shape. In some instances, the reinforcement member 400 can comprise one or more tether securement features associated therewith, including with the circumferential portion 406 of the reinforcement member 400.

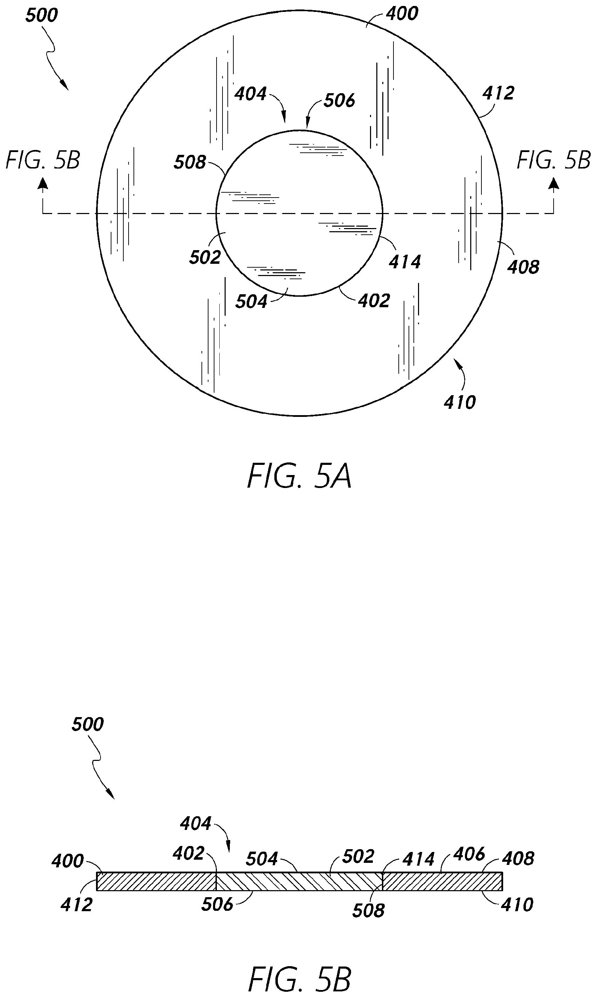

Figure 5A shows a top-down view, and Figure 5B shows a perspective view, of an example of a reinforced surgical pad device 500 comprising the reinforcement member 400 and a surgical pad 502. As described herein, the surgical pad 502 can be associated with the central portion 402 of the reinforcement member 400. The surgical pad 502 can be configured to be aligned with the opening 404 of the reinforcement member 400. Both the surgical pad 502 and the reinforcement member 400 can be configured to be aligned with the opening in the target tissue. For example, the surgical pad 502 can be positioned over the opening in the target tissue such that the surgical pad 502 can be aligned with the opening in the target tissue. The opening 404 of the reinforcement member 400 can be around the opening in the target tissue.

In some instances, the surgical pad 502 can be configured to be disposed within the opening 404 of the reinforcement member 400. The surgical pad 502 can be positioned over the opening in the target tissue such that a first surface 504 of the surgical pad 502 can be oriented toward the target tissue and/or opening, and a second surface 506 of the surgical pad 502 can be oriented away from the target tissue and/or opening. The reinforcement member 400 can be positioned to surround the surgical pad 502, the first surface 408 of the reinforcement member 400 being oriented toward the target tissue and the second surface 410 of the reinforcement member 400 being oriented away from the target tissue. The opening 404 of the reinforcement member 400 and the surgical pad 502 can comprise the same or different shape. In some instances, both the opening 404 of the reinforcement member 400 and the surgical pad 502 can comprise a circular shape. One or more lateral dimensions, such as a width and/or a length, of the surgical pad 502 can be the same as or shorter than that of the opening 404 of the reinforcement member 400. For example, a diameter of the surgical pad 502 can be less than or equal to that of the opening 404 of the reinforcement member 400. In some instances, the reinforced surgical pad device 500 can be provided as an integral device. The reinforcement member 400 can be integrated with the surgical pad 502. In some instances, the outer edge 508 of the surgical pad can be coupled to the inner edge 414 of the reinforcement member 400. In some instances, the surgical pad 502 and the reinforcement member 400 can be provided separately.

Alternatively, the reinforced surgical pad device 500 can comprise a surgical pad having a size larger than that of the opening 404 of the reinforcement member 400. An outer edge portion of the surgical pad can be configured to extend laterally beyond the inner edge 414 of the reinforcement member 400. For example, the outer edge portion of the surgical pad can be disposed between the reinforcement member 400 and the target tissue. One or more lateral dimensions, such as a width and/or a length, of the surgical pad can be longer than that of the opening 404 of the reinforcement member 400. For example, a diameter of the surgical pad can be longer than the diameter of the opening 404 of the reinforcement member 400. In some instances, the outer edge portion of the surgical pad can be associated with the reinforcement member 400, such as the first surface 408 of the reinforcement member 400. A second surface of the surgical pad outer edge portion can be coupled to the first surface 408 of the reinforcement member 400.

It will be understood that reinforcement members can comprise a number of different configurations and sizes. A reinforcement member can comprise a polygonal shape, such as a star shape, and/or a rectangular shape, including a square shape. In some instances, a size, such as a width and/or a length, of the reinforcement member can be selected based on patient anatomy.

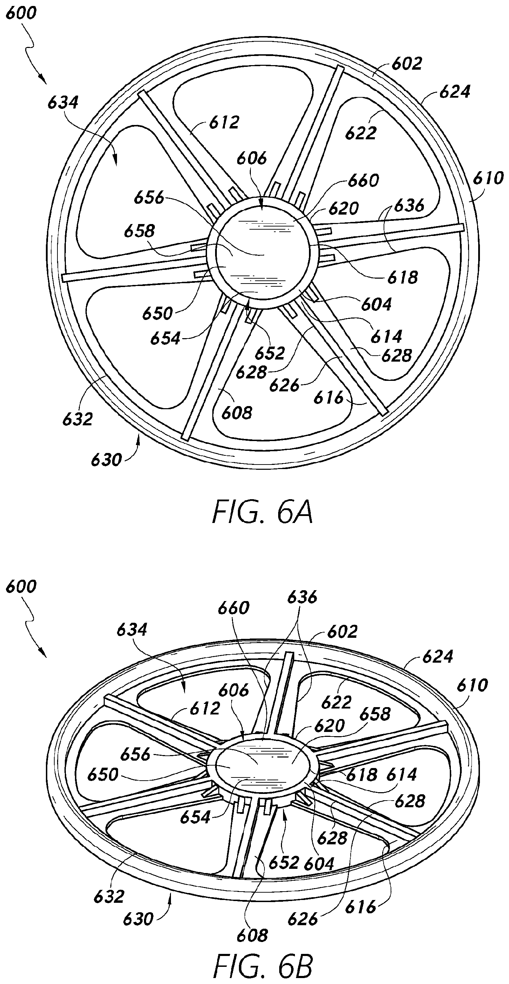

Figure 6A shows a top-down view, and Figure 6B shows a perspective view, of a reinforced surgical pad device 600 that includes a reinforcement member 602 and a surgical pad 650, where a circumferential portion 608 of the reinforcement member 602 comprises a plurality of radially arranged elongate portions 612. The reinforcement member 602 can comprise a central portion 604 defining an opening 606 extending therethrough. The surgical pad 650 can be associated with the central portion 604 of the reinforcement member 602. The circumferential portion 608 can extend around the central portion 604. The circumferential portion 608 can comprise an outer circumferential edge portion 610 and a plurality of elongate portions 612 extending between the central portion 604 and the outer circumferential edge portion 610. The plurality of elongate portions 612 can be arranged in a radial pattern to couple the central portion 604 and the outer circumferential edge portion 610 to one another. The reinforced surgical pad device 600 can be positioned over a target tissue such that the surgical pad 650 can be over an opening in the target tissue. The reinforcement member 602 can be over the target tissue such that the opening 606 of the reinforcement member 602 is positioned around the opening in the target tissue. Respective first surfaces 630, 652 of the reinforcement member 602 and surgical pad 650 can be oriented toward the target tissue and respective second surfaces 632, 654 of the reinforcement member 602 and surgical pad 650 can be oriented away from the target tissue.

In some instances, the plurality of elongate portions 612 can extend radially from the central portion 604 of the reinforcement member 602. Each of the plurality of elongate portions 612 can comprise a first end portion 614 oriented toward the central portion 604 and a second end portion 616 oriented away from the central portion 604. In some instances, the first end portion 614 can be adjacent to the central portion 604. In some instances, the second end portion 616 can be adjacent to the outer circumferential edge portion 610. For example, the plurality of elongate portions 612 can extend radially from the central portion 604 to the outer circumferential edge portion 610. The plurality of elongate portions 612 can be arranged radially between the central portion 604 and the outer circumferential edge portion 610 to couple the central portion 604 and the circumferential edge portion 610 to one another.

Although the circumferential portion 608 of the reinforcement member 602 is shown as comprising six elongate portions 612, it will be understood that a circumferential portion can comprise more or fewer elongate portions, such as four or eight. In some instances, the elongate portions 612 can be evenly spaced around the central portion 604. For example, an angle formed by adjacent elongate portions 612 of the reinforcement member 602 can be about 60°.

In some instances, the central portion 604 can comprise a ring-shaped portion. In some instances, the plurality of elongate portions 612 extend radially from the ring-shaped portion. The central portion 604 can comprise an inner edge 618 and an outer edge 620. The inner edge 618 can define the opening 606 of the reinforcement member 602. In some instances, the inner edge 618 can comprise a circular shape. It will be understood that other shapes can also be suitable, including for example, an oval shape and/or a polygonal shape, including a rectangular shape. The outer edge 620 can comprise a plurality of segments. For example, each segment can extend between respective adjacent elongate portions 612. In some instances, the outer edge 620 can comprise a plurality of segments of a circle. For example, the central portion 604 can comprise an annular shape.

In some instances, the outer circumferential edge portion 610 can comprise a ring-shaped portion. The outer circumferential edge portion 610 can comprise an inner edge 622 and an outer edge 624. The inner edge 622 can be oriented toward the central portion 604 and the outer edge 624 can be oriented away from the central portion 604. In some instances, the outer edge 624 can comprise a circular shape. It will be understood that other shapes can also be suitable, including for example, an oval shape and/or a polygonal shape, including a rectangular shape. The inner edge 622 can comprise a plurality of segments. For example, each segment can extend between respective adjacent elongate portions 612, such as between second end portions 616 of respective elongate portions 612. In some instances, the inner edge 622 can comprise a plurality of segments of a circle. For example, the outer circumferential edge portion 610 can comprise an annular shape. In some instances, the reinforcement member 602 can comprise a hub-and-spoke wheel configuration.

In some instances, the reinforcement member 602 can comprise one or more portions having a thickness different from one or more other portions. The one or more of the elongate portions 612 can comprise a longitudinal portion 626 along a longitudinal dimension. The longitudinal portion 626 can have a thickness thicker than adjacent portions 628 that are adjacent to the longitudinal portion 626 along the longitudinal dimension. The longitudinal dimension of an elongate portion 612 can extend between the first and second end portions 612, 614. In some instances, each of the plurality of elongate portions 612 can comprise a longitudinal portion 626 having a thickness thicker than adjacent portions 628. A thickness of an elongate portion 612 can be a dimension extending between opposing portions of the first and second surfaces 630, 632 of the reinforcement member 602. The thickness of the longitudinal portion 626 can provide additional structural support for the elongate portions 612. In some instances, a longitudinal portion 626 can extend along an entire or substantially entire longitudinal dimension, such as a length, of the respective elongate portion 612. For example, the longitudinal portion 626 can extend from the central portion 604 to the outer circumferential edge portion 610.

In some instances, the central portion 604 and/or the outer circumferential edge portion 610 can comprise one or more portions having a thickness thicker than one or more adjacent portions of the reinforcement member 602, including adjacent portions of the central portion 604 and/or adjacent portions of the outer circumferential edge portion 610. For example, the ring-shaped portion of the central portion 604 and/or the ring-shaped portion of the outer circumferential edge portion 610 can have a thickness thicker than one or more adjacent portions of the reinforcement member 602. In some instances, the ring-shaped portion of the central portion 604 can have a thickness thicker than one or more portions of the central portion 604 adjacent to the ring-shaped portion. In some instances, the ring-shaped portion of the outer circumferential edge portion 610 can have a thickness thicker than one or more portions of the outer circumferential edge portion 610 adjacent to the ring-shaped portion.

A lateral dimension, such as a width, of one or more of the elongate portions 612 can vary along a longitudinal dimension of the respective elongate portion 612. The lateral dimension can be perpendicular or substantially perpendicular to the longitudinal dimension and the thickness. In some instances, each of the plurality of elongate portions 612 can have a first end portion 614 having a width wider than that of a second end portion 616.

In some instances, the plurality of elongate portions 612 can comprise the same features. In some instances, six identical elongate portions 612 can be evenly spaced around the central portion 604. The circumferential portion 608 can comprise six openings 634 extending therethrough, each opening 634 defined by lateral edges 636 of respective adjacent elongate portions 612, a respective segment of the outer edge 620 of the central portion 604 and a respective segment of the inner edge 622 of the outer circumferential edge portion 610. In some instances, six identical elongate portions 612 can extend from the central portion 604 to the outer circumferential edge portion 610 to form six openings 634 having the same size and shape.

The surgical pad 650 can be configured to be associated with the central portion 604. In some instances, the surgical pad 650 can be configured to be associated with the inner edge 618 of the central portion 604 defining the opening 606 of the reinforcement member 602. For example, the surgical pad 650 can be positioned over the opening in the target tissue such that the surgical pad 650 can be aligned with the opening in the target tissue. The opening 606 of the reinforcement member 602 can be around the opening in the target tissue. In some instances, the surgical pad 650 can be configured to be disposed within the opening 606 of the reinforcement member 602. In some instances, both the opening 606 of the reinforcement member 602 and the surgical pad 650 can comprise a circular shape. Alternatively, the opening 606 and the surgical pad 650 can comprise different shapes. One or more lateral dimensions, such as a width and/or a length, of the surgical pad 650 can be the same as or shorter than that of the opening 606 of the reinforcement member 602. For example, a diameter of the surgical pad 650 can be less than or equal to that of the opening 606 of the reinforcement member 602. In some instances, the reinforcement member 602 can be integrated with the surgical pad 650, for example, the outer edge 660 of the surgical pad 650 can be coupled to the inner edge 618 of the central portion 604 of the reinforcement member 602. In some instances, the diameter of the surgical pad 650 can be equal to that of the opening 606 of the reinforcement member 602 and the surgical pad 650 can be coupled to the inner edge 618 of the central portion 604. Alternatively, the surgical pad 650 and the reinforcement member 602 can be provided separately.

In some instances, the reinforced surgical pad device 600 can comprise a surgical pad having a size larger than that of the opening 606 of the reinforcement member 602. A width and/or a length, such as a diameter, of the surgical pad can be longer than that of the opening 606. An outer edge portion of the surgical pad can be configured to extend laterally beyond the inner edge 618 of the central portion 604 of the reinforcement member 602. The outer edge portion of the surgical pad can be disposed between the reinforcement member 602 and the target tissue. In some instances, a second surface of the outer edge portion of the surgical pad can be associated with the first surface 630 of the reinforcement member 602.

Referring to Figure 6B, a perspective view of the reinforced surgical pad device 600 is shown. Portions of the reinforcement member 602 having different thicknesses are shown in more detail in Figure 6B. The second surface 632 of the reinforcement member 602 is shown as comprising features having different thicknesses. The first surface 630 of the reinforcement member can be smooth or substantially smooth. As described herein, the first surface 630 of the reinforcement member 602 can be configured to be oriented toward the target tissue. The smooth or substantially smooth surface can reduce or prevent irritation to the target tissue.

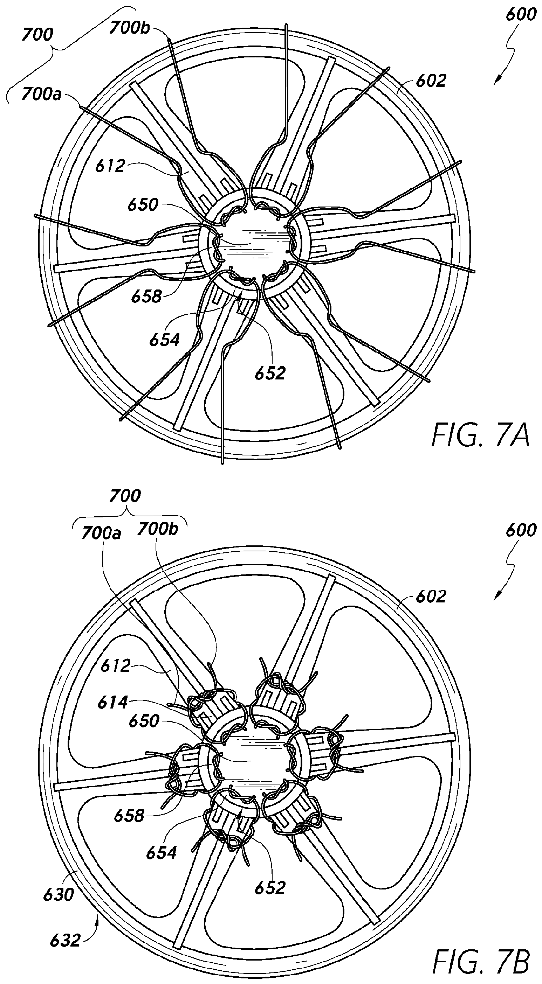

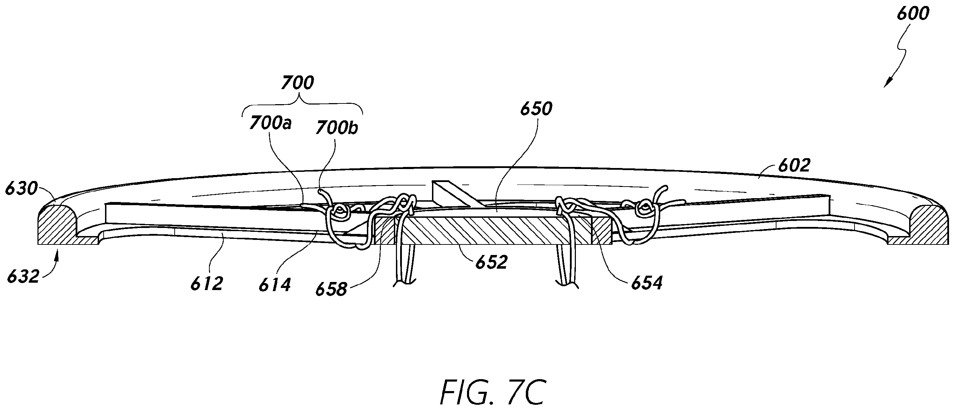

Figures 7A, 7B and 7C show a plurality of tethers 700 coupled to the reinforced surgical pad device 600 described with reference to Figure 6. Figure 7A is a top-down view showing a plurality of tethers 700 coupled to the surgical pad 650. Figure 7B is a top-down view, and Figure 7C is a side cross-sectional view, of the plurality of tethers 700 coupled to both the surgical pad 650 and the reinforcement member 602. Referring to Figure 7A, six tethers 700 are shown as being coupled to the surgical pad 650. In some instances, the reinforced surgical pad device 600 can be positioned over the target tissue such that the surgical pad 650, such as a central portion 656 of the surgical pad 650, is over the opening in the target tissue. Respective portions of the six tethers 700 can extend from the opening in the target tissue. Each tether 700 can comprise a pair of tether tails 700a, 700b, such that respective portions of each pair of tether tails 700a, 700b extending externally of the opening in the target tissue can be coupled to a corresponding portion of the surgical pad 650. The six pairs of tether tails 700a, 700b can be coupled to six respective portions of the surgical pad 650, such as at positions around an outer edge portion 658 of the surgical pad 650. Coupling the tether tails 700a, 700b can be achieved using any number of methods. In some instances, each pair of tether tails 700a, 700b can be coupled to a location on the surgical pad 650 configured to be adjacent or proximate to a respective elongate portion 612 of the reinforcement member 602. In some instances, securing each pair of tether tails 700a, 700b to a respective position on the surgical pad 650 can comprise forming knots using the pair tether tails 700a, 700b. Respective portions of each tether tail 700a, 700b extending externally from the opening in the target tissue can be advanced through the surgical pad 650, such as from the first surface 652 to the second surface 654 of the surgical pad 650. The tether tails 700a, 700b can then be knotted together over the second surface 654 of the surgical pad 650.

Referring to Figure 7B, the plurality of tethers 700 can be coupled to both the surgical pad 650 and the reinforcement member 602. For example, the plurality of tethers 700 can be secured to the surgical pad 650 as described with reference to Figure 7A. The tethers 700 can subsequently be secured to the reinforcement member 602 using any number of methods. In some instances, securing the plurality of tethers 700 to the reinforcement member 602 can comprise securing each pair of tether tails 700a, 700b to a respective elongate portion 612. In some instances, tethers tails 700a, 700b can be wound around a respective elongate portion 612. For example, portions of the tether tails 700a, 700b can be extended from the second surface 632 to the first surface 630 and back to the second surface 632 of the reinforcement member 602. The tether tails 700a, 700b can be knotted together over the second surface 632 of the reinforcement member 602.

Figure 7C is a side cross-sectional view showing the plurality of tethers 700 coupled to both the surgical pad 650 and the reinforcement member 602. Respective portions of a pair of tether tails 700a, 700b is shown as extending through the surgical pad 650 from the first surface 652 to the second surface 654 of the surgical pad 650. The pair of tether tails 700a, 700b are shown as being wound around an elongate portion 612, from the second surface 632 to the first surface 630 and back to the second surface 632 of the elongate portion 612.

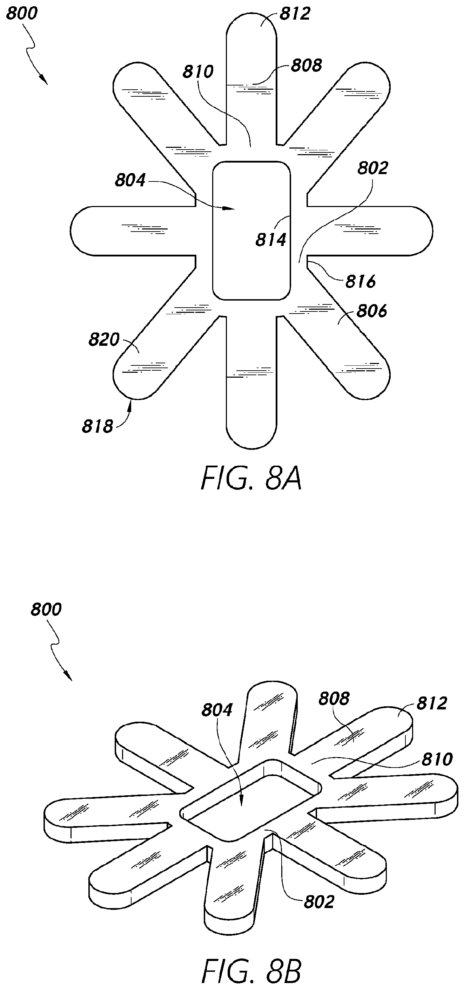

Figure 8A shows a top-down view, and Figure 8B shows a perspective view, of a reinforcement member 800 comprising a central portion 802 and a circumferential portion 806 around the central portion 802, where the circumferential portion 806 comprises a plurality of elongate portions 808. The plurality of elongate portions 808 can be arranged in a radial pattern. For example, the plurality of elongate portions 808 can be in a radial pattern around the central portion 802. The reinforcement member 800 can comprise a first surface 818 configured to be oriented toward a target tissue and a second surface 820 configured to be oriented away from the target tissue.

In some instances, the plurality of elongate portions 808 can extend radially from the central portion 802 of the reinforcement member 800. Each of the plurality of elongate portions 808 can comprise a first end portion 810 oriented toward the central portion 802 and a second end portion 812 oriented away from the central portion 802. In some instances, the plurality of elongate portions 808 can extend radially from the central portion 802 such that the first end portion 810 can be adjacent to the central portion 802.

Although the circumferential portion 806 of the reinforcement member 800 is shown as comprising eight elongate portions 808, it will be understood that a circumferential portion can comprise more or fewer elongate portions, such as four or six. In some instances, the elongate portions 808 can be evenly spaced around the central portion 802. For example, an angle formed by adjacent elongate portions 808 can be about 80°.

The central portion 802 of the reinforcement member 800 can define an opening 804 extending therethrough. In some instances, the central portion 802 can comprise a ring-shaped portion. The central portion 802 can comprise an inner edge 814 and an outer edge 816. In some instances, the plurality of elongate portions 808 can extend radially from the ring-shaped portion, including from the outer edge 816. The inner edge 814 can define the opening 804. In some instances, the inner edge 814 can comprise a polygonal shape, such as a rectangle. It will be understood that other shapes can also be suitable, including for example, an oval shape and/or circular shape. The outer edge 816 can comprise a plurality of segments. For example, each segment can extend between respective adjacent elongate portions 808. In some instances, the outer edge 816 can comprise a plurality of segments of a rectangle.

Figure 8B shows a perspective view of the reinforcement member 800. In some instances, the reinforcement member 800 can have a uniform thickness. For example, the central portion 802 and the plurality of elongate portions 808 can have the same thickness. In some instances, each of the plurality of elongate portions 808 can comprise a uniform thickness, for example having a uniform thickness along both a longitudinal dimension and a lateral dimension. The thickness can be a dimension extending between opposing portions of the first surface 818 and the second surface 820 of the elongate portion 808. The longitudinal dimension can extend between the first end portion 810 and second end portion 812. The lateral dimension can be a dimension perpendicular or substantially perpendicular to the longitudinal dimension and the thickness.

In some instances, each of the plurality of elongate portions 808 can comprise a uniform or substantially uniform lateral dimension along the longitudinal dimension. For example, each elongate portion 808 can have a uniform width along the length of the elongate portion 808. Alternatively, the lateral dimension of one or more of the elongate portions 808 can be non-uniform along the longitudinal dimension. For example, the first end portion 810 of each of the plurality of elongate portions 808 can have a width wider than that of the second end portion 812.

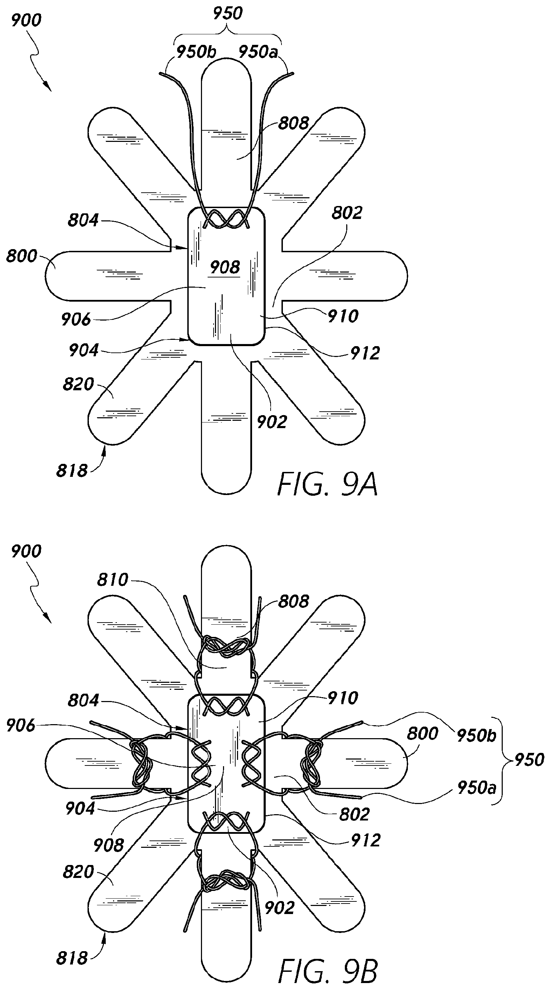

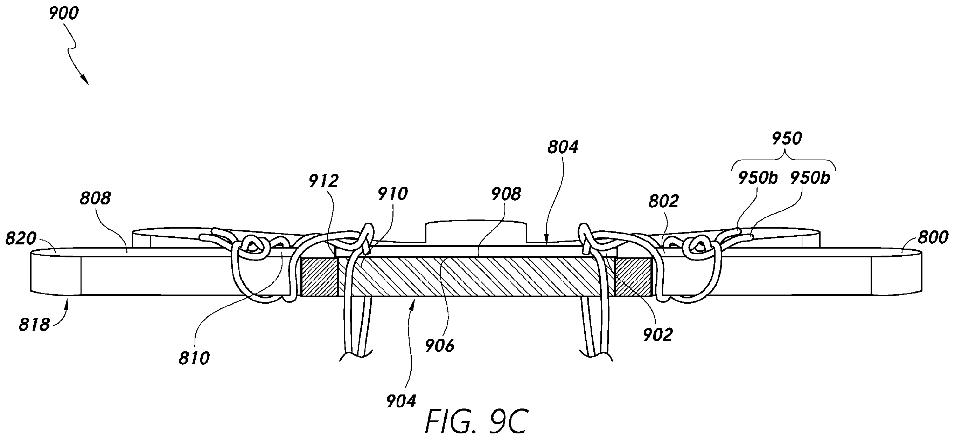

Figures 9A, 9B and 9C show various views of a reinforced surgical pad device 900 comprising the reinforcement member 800 described with reference to Figure 8, and a surgical pad 902. Figure 9A is a top-down view of the reinforced surgical pad device 900 and a tether 950 coupled to the surgical pad 902. Figure 9B is a top-down view, and Figure 9C is a side cross-sectional view, showing additional tethers 950 coupled to the surgical pad 902. The plurality of tethers 950 can be coupled to both the surgical pad 902 and the reinforcement member 800.

The surgical pad 902 can be associated with the central portion 802 of the reinforcement member 800. The surgical pad 902 can have a first surface 904 configured to be oriented toward a target tissue and a second surface 906 configured to be oriented away from the target tissue. A central portion 908 of the surgical pad 902 can be configured to be positioned over an opening in the target tissue. The surgical pad 902 can be configured to be disposed within the opening 804 defined by the central portion 802 of the reinforcement member 800. In some instances, the surgical pad 902 can be coupled to the central portion 802 of the reinforcement member 802, such as the inner edge 814 of the central portion 802 defining the opening 804. The surgical pad 902 can be positioned over an opening in the target tissue, for example such that a central portion 908 of the surgical pad 902 is over the opening, and the inner edge 814 defining the opening 804 of the reinforcement member 800 is over the target tissue and around the opening in the target tissue. The surgical pad 902 and the opening 804 of the reinforcement member 800 can be aligned with the opening in the target tissue. In some instances, the surgical pad 902 can have a shape the same as or similar to that of the opening 804 defined by the central portion 802 of the reinforcement member 800. For example, an outer edge 912 of the surgical pad 902 can have a rectangular shape. In some instances, the surgical pad 902 can have a size the same as or similar to that of the opening 804 of the reinforcement member 800. For example, the outer edge 912 of the surgical pad 902 can be configured to be associated with, such as being coupled to, the inner edge 814 of the central portion 802.

Referring to Figure 9A, a tether 950 is shown as being coupled to the surgical pad 902. Each tether 950 can comprise a pair of tether tails 950a, 950b. Respective portions of the tether tails 950a, 950b can extend from the opening in the target tissue. In some instances, securing each pair of tether tails 950a, 950b to a respective position on the surgical pad 902 can comprise forming a knot with the pair of tether tails 950a, 950b. Respective portions of each tether tail 950a, 950b extending externally from the opening in the target tissue can be advanced through the surgical pad 902, such as from the first surface 904 to the second surface 906 of the surgical pad 902. A knot can be formed using the tether tails 950a, 950b over the second surface 906. The tether tails 950a, 950b can be coupled to an outer edge portion 910 of the surgical pad 902. For example, while the surgical pad 902 is received within the opening 804 of the reinforcement member 800, the tether tails 950a, 950b can be coupled to a location on the outer edge portion 910 of the surgical pad 902 configured to be adjacent or proximate to a respective elongate portion 808 of the reinforcement member 800.

Referring to Figure 9B, additional tethers 950 can be coupled to the surgical pad 902 such that four tethers 950, each comprising a pair of tether tails 950a, 950b, are coupled to the surgical pad 902. The tethers 950 can be coupled to both the surgical pad 902 and the reinforcement member 800. For example, the plurality of tethers 950 can be secured to the surgical pad 902 as described with reference to Figure 9A. The tethers 950 can then be secured to the reinforcement member 800 using any number of methods. In some instances, securing the plurality of tethers 950 to the reinforcement member 800 can comprise securing each pair of tether tails 950a, 950b to a respective elongate portion 810. For example, each pair of tethers tail 950a, 950b can be wound around a respective elongate portion 808 and subsequently knotted together. A knot can be formed using a pair of tether tails 950a, 950b over a second surface 820 of a first end portion 810.

Figure 9C is a side cross-sectional view of the reinforced surgical pad device 900 shown in Figure 9B. Figure 9C shows the plurality of tethers 950 coupled to both the surgical pad 902 and the reinforcement member 800. Respective portions of a pair of tether tails 950a, 950b is shown as extending through the surgical pad 902 from the first surface 904 to the second surface 906 of the surgical pad 902. The pair of tether tails 950a, 950b are shown as being wound around an elongate portion 808, from the second surface 820 to the first surface 818 and back to the second surface 820 of the elongate portion 808. The tether tails 950a, 950b can be knotted together over the second surface 820 of the reinforcement member 800. For example, each pair of tether tails 950a, 950b can be knotted to itself over the second surface 820 to secure the tether 950 to the reinforcement member 800.

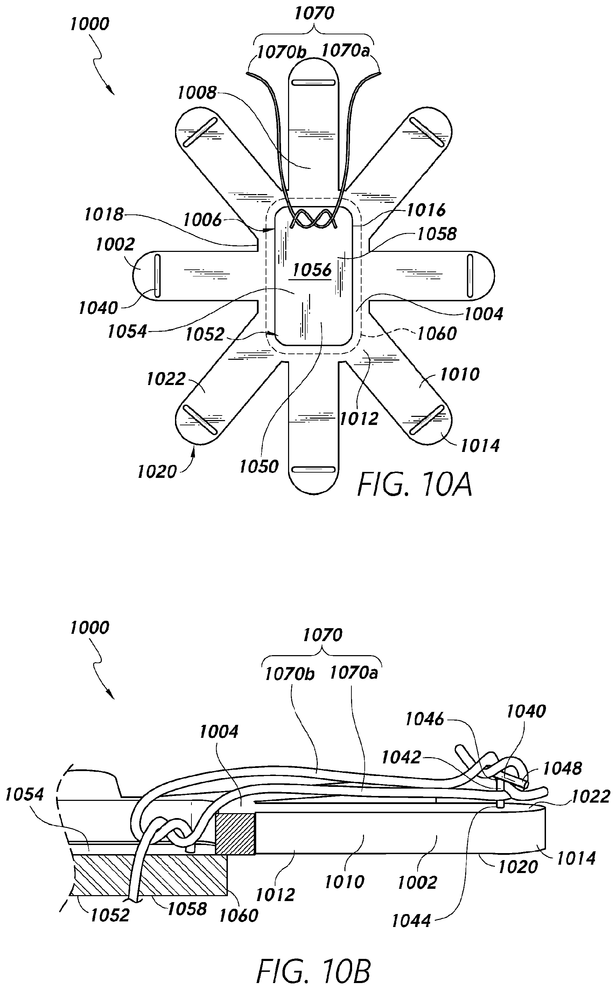

Figure 10A is a top-down view, and Figure 10B is a side cross-sectional view, of an example of a reinforced surgical pad device 1000 that includes a reinforcement member 1002 and a surgical pad 1050, where the reinforcement member 1002 can comprise a plurality of tether securement features 1040 associated with a respective one of a plurality of elongate portions 1010 of the reinforcement member 1002. Referring to Figure 10A, the reinforcement member 1002 can comprise a central portion 1004, and a circumferential portion 1008 around the central portion 1004, the circumferential portion 1008 comprising the plurality of elongate portions 1010 arranged in a radial pattern. Each elongate portion 1010 can comprise a first end portion 1012 oriented toward the central portion 1004 and a second end portion 1014 oriented away from the central portion 1004. A tether securement feature 1040 can be associated with the second end portion 1014 of a respective elongate portion 1010. In some instances, the tether securement feature 1040 can be coupled to the second end portion 1014. Alternatively, the tether securement feature 1040 can extend from and be integrated with the second end portion 1014.

The surgical pad 1050 can be configured to be associated with the central portion 1004 of the reinforcement member 1002. The surgical pad 1050 can have a first surface 1052 configured to be oriented toward a target tissue and a second surface 1054 configured to be oriented away from the target tissue. A tether 1070 is shown as being coupled to the surgical pad 1050, such as at a location adjacent or proximate to a respective elongate portion 1010 of the reinforcement member 1002. Respective portions of each tether tail 1070a, 1070b of the tether 1070 extending externally from the opening in the target tissue can be advanced through the surgical pad 1050, such as from the first surface 1052 to the second surface 1054 of the surgical pad 1050. A knot can be formed over the second surface 1054 of the surgical pad 1050 using the pair of tether tails 1070a, 1070b. As described in further detail herein, a tether securement feature 1040 can be configured to allow coupling thereto of a respective pair of tether tails 1070a, 1070b such that the tether 1070 can be coupled to both the surgical pad 1050 and the reinforcement member 1002.

In some instances, the surgical pad 1050 can have a size larger than that of the opening 1006 of the reinforcement member 1002. A central portion 1056 of the surgical pad 1050 can be configured to be positioned over an opening in the target tissue. An outer edge portion 1058 can be configured to be associated with the central portion 1004 of the reinforcement member 1002. For example, the outer edge portion 1058 of the surgical pad 1050 can be configured to be coupled to the central portion 1004 of the reinforcement member 1002. A portion of the surgical pad 1050 can be configured to be disposed under the opening 1006 defined by the central portion 1004. For example, the surgical pad 1050 can be positioned over the target tissue and the reinforcement member 1002 can be positioned partially over the surgical pad 1050 such that the outer edge portion 1058 of the surgical pad 1050 is between the reinforcement member 1002, such as a first surface 1020 of the reinforcement member 1002, and the target tissue. In some instances, an outer edge 1060 of the surgical pad 1050 can be configured to be positioned between the reinforcement member 1002, such as the central portion 1004 of the reinforcement member 1002, and the target tissue. The first surface 1020 of the central portion 1004 can comprise at least a portion configured to be coupled to at least a portion of the second surface 1054 of the outer edge portion 1058 of the surgical pad 1050. The opening 1006 of the reinforcement member 1002 can be over the opening in the target tissue such that an inner edge 1016 of the central portion 1004 can be around the opening in the target tissue. The surgical pad 1050 and the opening 1006 of the reinforcement member 1002 can be aligned with the opening in the target tissue.

The surgical pad 1050 may or may not have a shape the same as or similar to that of the opening 1006 defined by the central portion 1004 of the reinforcement member 1002. In some instances, both the opening 1006 defined by the central portion 1004 and the outer edge 1060 of the surgical pad 1050 can have a rectangular shape.

Referring to Figure 10B, the tether securement feature 1040 can comprise a "T" shape. A vertical portion 1042 can extend out of the second surface 1022 and be perpendicularly or substantially perpendicularly to the second surface 1022 of the elongate portion 1010, having a first end 1044 oriented toward the elongate portion 1010 and a second end 1046 oriented away from the elongate portion 1010. A horizontal portion 1048 can be associated with, such as being coupled to or integrated with, the second end 1046 of the vertical portion 1042. Securing a pair of tether tails 1070a, 1070b to the tether securement feature 1040 can comprise winding portions of the pair of tether tails 1070a, 1070b around the tether securement feature 1040, including around the vertical portion 1042 and/or the horizontal portion 1048. A knot can be formed using the pair of tether tails 1070a, 1070a after a desired portion of the tether tails 1070a, 1070b have been wound around the tether securement feature 1040.

As shown in Figure 10B, the outer edge portion 1058 of the surgical pad 1050 can be configured to be beneath the central portion 1004 of the reinforcement member 1002. For example, the outer edge portion 1058 of the surgical pad 1050 can be between the reinforcement member 1002 and the target tissue.

The reinforcement member 1002 can have one or more other features of the reinforcement member 800 described with reference to Figures 8 and 9. For example, the circumferential portion 1008 can comprise eight elongate portions 1010 extending radially from the central portion 1004. The elongate portions 1010 can be evenly spaced around the central portion 1004. The central portion 1004 can comprise a ring-shaped portion. The central portion 1004 can comprise an inner edge 1016 and an outer edge 1018. The inner edge 1016 can define an opening 1006 comprising a rectangular shape. The outer edge 1018 can comprise a plurality of segments of a rectangle, each segment extending between respective adjacent elongate portions 1010. The reinforcement member 1002 can have a uniform thickness. In some instances, each of the plurality of elongate portions 1010 can comprise a uniform lateral dimension along a longitudinal dimension of the respective elongate portion 1010. For example, each elongate portion 1010 can have a uniform width along the length of the elongate portion 1010.

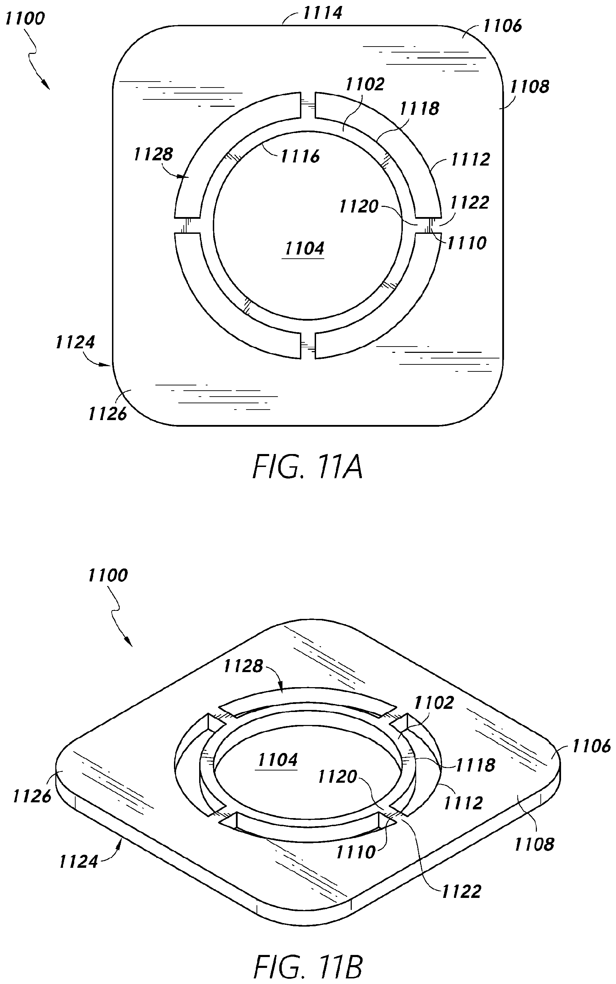

Figure 11A shows a top-down view, and Figure 11B shows a perspective view, of an example of a reinforcement member 1100 comprising an outer edge 1114 having a rectangular shape. The reinforcement member 1100 can comprise a central portion 1102, and a circumferential portion 1106 around the central portion 1102, the circumferential portion 1106 comprising an outer circumferential edge portion 1108 and a plurality of elongate portions 1110 arranged in a radial pattern between the central portion 1102 and the outer circumferential edge portion 1108. An outer edge 1114 of the outer circumferential edge portion 1108 can have a rectangular shape, including a square shape. A first surface 1124 of the reinforcement member 1100 can be configured to be oriented toward a target tissue and a second surface 1126 of the reinforcement member 1100 can be configured to be oriented away from the target tissue.

In some instances, the outer circumferential edge portion 1108 can comprise a ring-shaped portion. The outer circumferential edge portion 1108 can comprise an inner edge 1112 oriented toward the central portion 1102 and the outer edge 1114 oriented away from the central portion 1102. In some instances, the outer edge 1114 can comprise a polygonal shape, including a square shape. It will be understood that other shapes can also be suitable, including for example, shapes comprising one or more curved sides, such as a circular and/or an oval shape. The inner edge 1112 can comprise a plurality of segments. For example, each segment can extend between respective adjacent elongate portions 1110. In some instances, the inner edge 1112 can comprise a plurality of segments of a circle.

The central portion 1102 can define an opening 1104 extending therethrough. In some instances, the central portion 1102 can comprise a ring-shaped portion. In some instances, the plurality of elongate portions 1110 can be arranged in a radial pattern between the ring-shaped portion and the outer circumferential edge portion 1108. The plurality of elongate portions 1110 can extend radially from the ring-shaped portion. The central portion 1102 can comprise an inner edge 1116 and an outer edge 1118. The inner edge 1116 can define the opening 1104 of the reinforcement member 1100. In some instances, the inner edge 1116 of the central portion 1102 can comprise a circular shape. It will be understood that other shapes can also be suitable, including for example, an oval and/or a polygonal shape, such as a rectangular shape. The outer edge 1118 can comprise a plurality of segments. For example, each segment can extend between respective adjacent elongate portions 1110. In some instances, the outer edge 1118 can comprise a plurality of segments of a circle. For example, the central portion 1102 can comprise an annular shape.

Each of the plurality of elongate portions 1110 comprise a first end portion 1120 oriented toward the central portion 1102 and a second end portion 1122 oriented away from the central portion 1102. The plurality of elongate portions 1110 can be arranged in a radial pattern to couple the central portion 1102 and the outer circumferential edge portion 1108. In some instances, the first end portion 1120 can be adjacent to the central portion 1102. In some instances, the second end portion 1122 can be adjacent to the outer circumferential edge portion 1108. For example, the plurality of elongate portions 1110 can extend radially from the central portion 1102 to the outer circumferential edge portion 1108. The plurality of elongate portions 1110 can extend from the outer edge 1118 of the ring-shaped central portion to the inner edge 1112 of the outer circumferential edge portion 1108. The first end portion 1120 of each elongate portion 1110 can be adjacent to respective segments of the outer edge 1118 of the central portion 1102. The second end portion 1122 of each elongate portion 1110 can be adjacent to respective segments of the inner edge 1116 of the outer circumferential edge portion 1108.

Although the circumferential portion 1106 of the reinforcement member 1100 is shown as comprising four elongate portions 1110, it will be understood that a circumferential portion can comprise more or fewer elongate portions, such as six or eight elongate portions. In some instances, the elongate portions 1110 can be evenly spaced around the central portion 1102. An angle formed by adjacent elongate portions 1110 of the reinforcement member 1100 can be about 90°.

Figure 11B is a perspective view of the reinforcement member 1100. In some instances, the reinforcement member 1100 can have a uniform thickness. For example, the central portion 1102, the outer circumferential edge portion 1108, and the elongate portions 1110 can have the same or similar thickness. In some instances, each of the plurality of elongate portions 1110 can comprise a uniform thickness, for example having a uniform thickness along both a longitudinal dimension and a lateral dimension. The thickness can be a dimension extending between opposing portions of the first surface 1124 and the second surface 1126 of the elongate portion 1110. The longitudinal dimension can extend between the first end portion 1120 and second end portion 1122 of the elongate portion 1110. The lateral dimension can be a dimension perpendicular or substantially perpendicular to both the longitudinal dimension and the thickness. In some instances, each of the plurality of elongate portions 1110 can comprise a uniform lateral dimension along the longitudinal dimension. Each elongate portion 1110 can have a uniform width along the length of the elongate portion 1110. Alternatively, the lateral dimension of one or more of the elongate portions 1110 can be non-uniform along the longitudinal dimension, the first end portion 1120 comprising a width wider than that of the second end portion 1122.

In some instances, a lateral dimension, such as a width, of the outer circumferential edge portion 1108 can be longer than the longitudinal dimension, such as a length, of an elongate portion 1110. The lateral dimension of the outer circumferential edge portion 1108 can be a dimension extending between two opposing positions of the inner edge 1112 and outer edge 1114 of the circumferential edge portion 1108.

The circumferential portion 1106 can comprise four openings 1128 extending therethrough, each opening 1128 defined by lateral edges of respective adjacent elongate portions 1110, a respective segment of the outer edge 1118 of the central portion 1102 and a respective segment of the inner edge 1112 of the outer circumferential edge portion 1108. In some instances, the plurality of elongate portions 1110 can comprise the same features and can be evenly spaced around the central portion 1102 such that the four openings 1128 can comprise the same size and shape.

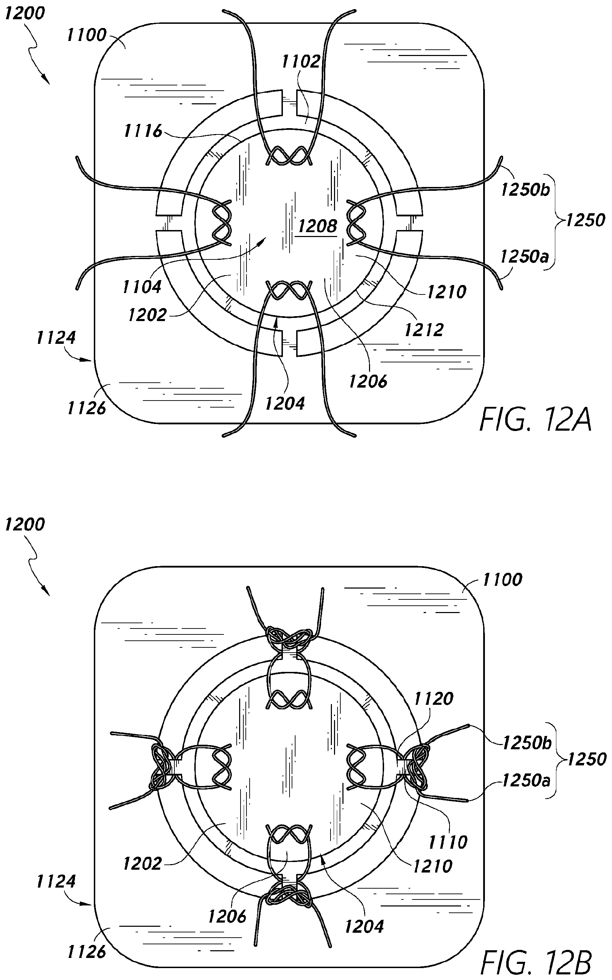

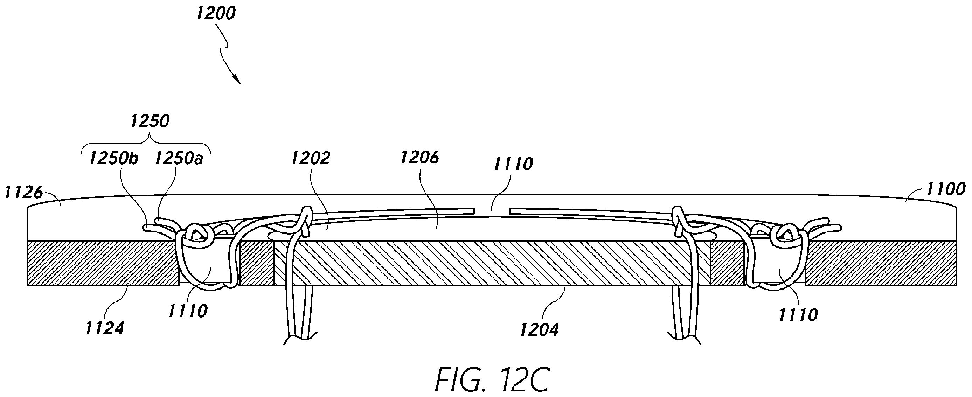

Figure 12A is a top-down view of a reinforced surgical pad device 1200 comprising the reinforcement member 1100 described with reference to Figure 11 and a surgical pad 1202 associated with the central portion 1102 of the reinforcement member 1100. Figure 12A shows a plurality of tethers 1250 coupled to the surgical pad 1202. Figure 12B is a top-down view, and Figure 12C is side cross-sectional view, showing the plurality of tethers 1250 coupled to the reinforcement member 1100 and the surgical pad 1202.

The surgical pad 1202 can have a first surface 1204 configured to be oriented toward a target tissue and a second surface 1206 configured to be oriented away from the target tissue. A central portion 1208 of the surgical pad 1202 can be configured to be positioned over an opening in the target tissue. The surgical pad 1202 can be configured to be disposed within the opening 1104 defined by the central portion 1102. The surgical pad 1202 can be positioned over an opening in the target tissue such that the opening 1104 of the reinforcement member 1100 is over and around the opening in the target tissue. The surgical pad 1202 and the opening 1104 of the reinforcement member 1100 can be aligned with the opening in the target tissue. In some instances, the surgical pad 1202 can have a shape the same as or similar to that of the opening 1104 defined by the central portion 1102 of the reinforcement member 1100. For example, an outer edge 1212 of the surgical pad 1202 can have a circular or substantially circular shape. In some instances, the surgical pad 1202 can have a size the same as or similar to that of the opening 1104 of the reinforcement member 1100. For example, the outer edge 1212 of the surgical pad 1202 can be configured to be associated with, such as coupled to, the inner edge 1116 of the central portion 1102.

Referring to Figure 12A, four tethers 1250 are shown being coupled to the surgical pad 1202. Respective portions of the four tethers 1250 can extend from the opening in the target tissue. Each tether 1250 can comprise a pair of tether tails 1250a, 1250b. Coupling the tether tails 1250a, 1250b can be achieved using any number of methods. Each pair of tether tails 1250a, 1250b can be coupled to an outer edge portion 1210 of the surgical pad 1202. For example, while the surgical pad 1202 is received within the opening 1104 of the reinforcement member 1100, the tether tails 1250a, 1250b can be coupled to a location on the outer edge portion 1210 of the surgical pad 1202 configured to be at a location adjacent or proximate to a respective elongate portion 1110 of the reinforcement member 1100. Respective portions of each tether tail 1250a, 1250b extending externally from the opening in the target tissue can be advanced through the surgical pad 1202, such as from the first surface 1204 to the second surface 1206 of the surgical pad 1202, including at corresponding positions in the outer edge portion 1210. In some instances, securing each pair of tether tails 1250a, 1250b to a respective position on the surgical pad 1202 can comprise forming a knot with a corresponding pair of tether tails 1250a, 1250b over the second surface 1206 of the surgical pad 1202.

Referring to Figure 12B, in some instances, the plurality of tethers 1250 can be coupled to both the surgical pad 1202 and the reinforcement member 1100. For example, the plurality of tethers 1250 can be secured to the surgical pad 1202 as described with reference to Figure 12A. The tethers 1250 can then be secured to the reinforcement member 1100 using any number of methods. In some instances, securing the plurality of tethers 1250 to the reinforcement member 1100 can comprise securing each pair of tether tails 1250a, 1250b to a respective elongate portion 1110. In some instances, securing each pair of tether tails 1250a, 1250b to a respective elongate portion 1110 can comprise forming a knot with a corresponding pair of tether tails 1250a, 1250b over the second surface 1126 of the elongate portion 1110. A pair of tether tails 1250a, 1250b can be wound around the corresponding elongate portion 1110. A knot can then be formed using the pair of tether tails 1250a, 1250b over the second surface 1126 of the elongate portion 1110.

Figure 12C is a side cross-sectional view of the reinforced surgical pad device 1200 shown in Figure 12B. Respective portions of a pair of tether tails 1250a, 1250b is shown as extending through the surgical pad 1202 from the first surface 1204 to the second surface 1206. Securing the pair of tether tails 1250a, 1250b to the surgical pad 1202 can comprise forming a knot over the second surface 1206 of the surgical pad 1202. The pair of tether tails 1250a, 1250b can then be wound around an elongate portion 1110 and knotted to secure the pair of tether tails 1250a, 1250b to the elongate portion 1110. The tether tails 1250a, 1250b can be extended from the first surface 1124 of the reinforcement member 1100 configured to be oriented toward the target tissue to the second surface 1126 of the reinforcement member 1100 configured to be oriented away the target tissue, and back to the second surface 1126. The knot can then be formed over the second surface 1126. The number of times the tether tails 1250a, 1250b are wound around the elongate portion 1110 can vary.

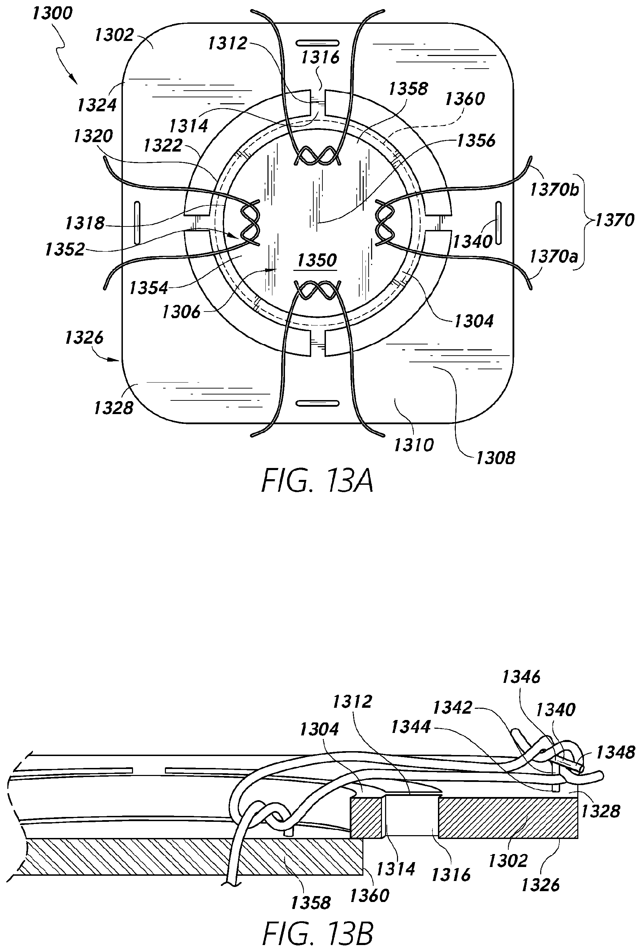

Figure 13A is a top-down view, and Figure 13B is a side cross-sectional view, of an example of a reinforced surgical pad device 1300 that can include a reinforcement member 1302 and a surgical pad 1350, wherein this example does not comprise all features of the claimed invention, where the reinforcement member 1302 can comprise a plurality of tether securement features 1340 associated with an outer circumferential edge portion 1310. Referring to Figure 13A, the reinforcement member 1302 can comprise a central portion 1304, and a circumferential portion 1308 around the central portion 1304, the circumferential portion 1308 comprising the outer circumferential edge portion 1310 and a plurality of elongate portions 1312 arranged in a radial pattern between the central portion 1304 and the outer circumferential edge portion 1310. The central portion 1304 can define an opening 1306 extending through the reinforcement member 1302. The reinforcement member 1302 can be positioned over a target tissue such that a first surface 1326 is oriented toward the target tissue and a second surface 1328 is oriented away from the target tissue. The plurality of tether securement features 1340 can be associated with the second surface 1328 of the outer circumferential edge portion 1310. For example, each of the plurality of tether securement features 1340 can be coupled to and/or extend from the second surface 1328 of the outer circumferential edge portion 1310.

Each of the plurality of elongate portions 1312 can comprise a first end portion 1314 oriented toward the central portion 1304 and a second end portion 1316 oriented toward the outer circumferential edge portion 1310. Each of the plurality of tether securement features 1340 can be disposed between a second end portion 1316 of a respective elongate portion 1312 and an outer edge 1324 of the outer circumferential edge portion 1310.

The surgical pad 1350 can be configured to be associated with the central portion 1304 of the reinforcement member 1302. For example, the surgical pad 1350 can be configured to be coupled to the central portion 1304 of the reinforcement member 1302. The surgical pad 1350 can have a first surface 1352 configured to be oriented toward a target tissue and a second surface 1354 configured to be oriented away from the target tissue. In some instances, the surgical pad 1350 can have a size larger than that of the opening 1306 of the reinforcement member 1302. A central portion 1356 of the surgical pad 1350 can be configured to be positioned over an opening in the target tissue. An outer edge portion 1358 of the surgical pad 1350 can be configured to be associated with the central portion 1304 of the reinforcement member 1302. A portion of the surgical pad 1350 can be configured to be disposed under the opening 1306 defined by the central portion 1304. For example, the surgical pad 1350 can be positioned over the target tissue and the reinforcement member 1302 can be positioned partially over the surgical pad 1350 such that the outer edge portion 1358 of the surgical pad 1350 is between the reinforcement member 1302, including the first surface 1326 of the reinforcement member 1302, and the target tissue. An outer edge 1360 of the surgical pad 1350 can be configured to be positioned between the central portion 1304 of the reinforcement member 1302 and the target tissue. An inner edge 1318 of the central portion 1304 defining the opening 1306 of the reinforcement member 1302 can be over the target tissue, and over and in contact with at least a portion of the outer edge portion 1358 of the surgical pad 1350. The inner edge 1318 can be around the opening in the target tissue. The surgical pad 1350 and the opening 1306 of the reinforcement member 1302 can be aligned with the opening in the target tissue.

The surgical pad 1350 may or may not have a shape the same as or similar to that of the opening 1306 defined by the central portion 1304 of the reinforcement member 1302. In some instances, the opening 1306 defined by the central portion 1304 and the outer edge 1360 of the surgical pad 1350 can have a circular or substantially circular shape.

Referring again to Figure 13A, four tethers 1370 are shown being coupled to the surgical pad 1350. Each tether 1370 can comprise a pair of tether tails 1370a, 1370b. For example, respective portions of the four pairs of tether tails 1370a, 1370b extending from the opening in the target tissue can be advanced through the surgical pad 1350, such as from the first surface 1352 to the second surface 1354 of the surgical pad 1350. In some instances, securing each pair of tether tails 1370a, 1370b to a respective position on the surgical pad 1350 can comprise forming a knot with a corresponding pair of the tether tails 1370a, 1370b. Respective portions of each tether tail 1370a, 1370b extending externally from the opening in the target tissue can be coupled to the surgical pad 1350 at a location adjacent or proximate to a respective elongate portion 1312 of the reinforcement member 1302.

Referring to Figure 13B, the surgical pad 1350 can be disposed partially below the reinforcement member 1302. For example, the outer edge portion 1358 of the surgical pad 1350 can be configured to be beneath the central portion 1304 of the reinforcement member 1302. The tether securement feature 1340 can comprise a "T" shape. For example, the tether securement feature 1340 can comprise a vertical portion 1342 can extend perpendicularly or substantially perpendicularly from the second surface 1328 of the elongate portion 1312, having a first end 1344 oriented toward the elongate portion 1312 and a second end 1346 oriented away from the elongate portion 1312. A horizontal portion 1348 can be associated with, such as coupled to or integrated with, the second end 1346 of the vertical portion 1342. Securing a pair of tether tails 1370a, 1370b to the tether securement feature 1340 can comprise winding the pair of tether tails 1370a, 1370b around the tether securement feature 1340, including around the vertical portion 1342 and/or the horizontal portion 1348. A knot can be formed using the pair of tether tails 1370a, 1370a after a desired portion of the tether tails 1370a, 1370b have been wound around the tether securement feature 1340.

The reinforcement member 1302 can have one or more other features of the reinforcement member 1100 described with reference to Figures 11 and 12. For example, the circumferential portion 1308 can comprise four elongate portions 1312 extending radially from the central portion 1304. The elongate portions 1312 can be evenly spaced around the central portion 1304. The central portion 1304 can comprise a ring-shaped portion. The central portion 1304 can comprise the inner edge 1318 and an outer edge 1320. The inner edge 1318 can define an opening 1306 comprising a circular shape. The outer edge 1320 can comprise a plurality of segments of a circle, each segment extending between respective adjacent elongate portions 1312. The reinforcement member 1302 can have a uniform thickness. In some instances, each of the plurality of elongate portions 1312 can comprise a uniform lateral dimension along the longitudinal dimension. For example, each elongate portion 1312 can have a uniform width along the length of the elongate portion 1312. In some instances, the outer circumferential edge portion 1310 can comprise a ring-shaped portion. The outer circumferential edge portion 1310 can comprise an inner edge 1322 oriented toward the central portion 1304 and the outer edge 1324 oriented away from the central portion 1304. In some instances, the outer edge 1324 can comprise a polygonal shape, including a square shape. It will be understood that other shapes can also be suitable, including for example, shapes comprising one or more curved sides, such as a circular and/or an oval shape. The inner edge 1322 can comprise a plurality of segments. For example, each segment can extend between respective adjacent elongate portions 1312. In some instances, the inner edge 1322 can comprise a plurality of segments of a circle. In some instances, the reinforcement member 1302 can comprise four identical elongate portions 1312 extending from the central portion 1304 to the outer circumferential edge portion 1310 to form four openings having the same size and shape

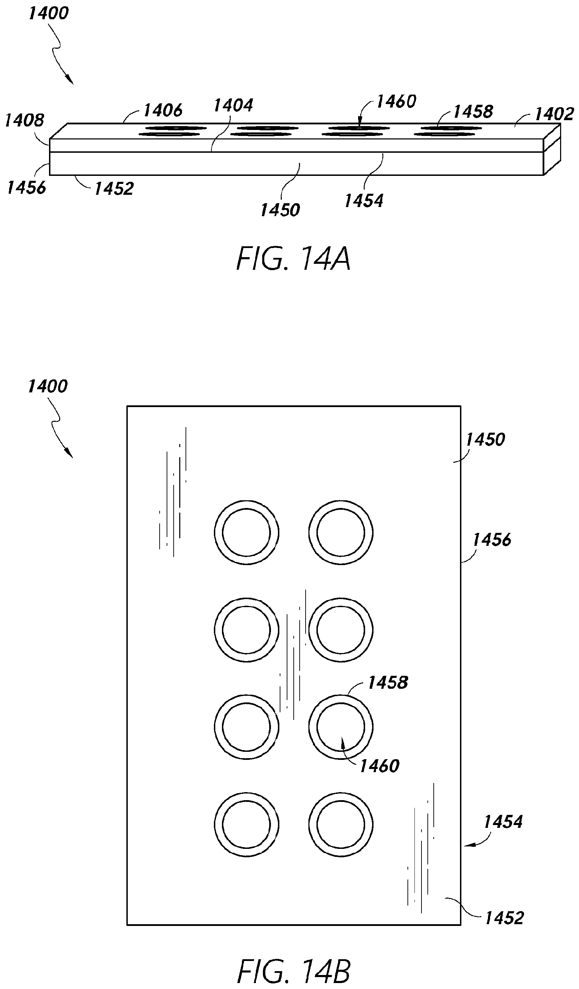

Figure 14A is a perspective view, and Figure 14B is a top-down view, of an example of a reinforced surgical pad device 1400 comprising a reinforcement member 1402 and a surgical pad 1450 having a plurality of pre-formed openings 1460 extending therethrough. This example does not comprise all features of the claimed invention. The plurality of pre-formed openings 1460 can facilitate threading therethrough of tethers. The reinforced surgical pad device 1400 can be positioned over an opening in a target tissue such that a first surface 1404 of the reinforcement member 1402, and a first surface 1452 of the surgical pad 1450, can be oriented toward the target tissue. A second surface 1406 of the reinforcement member 1402, and a second surface 1454 of the surgical pad 1450, can be oriented away from the target tissue. The surgical pad 1450 can be configured to be associated with the first surface 1404 of the reinforcement member 1402 such that the surgical pad 1450 can be between the reinforcement member 1402 and the target tissue while the reinforced surgical pad device 1400 is positioned over the target tissue. The reinforcement member 1402 can comprise a plurality of openings at locations corresponding to those of the plurality of pre-formed openings 1460 of the surgical pad 1450. While the surgical pad 1450 is coupled to the first surface 1404 of the reinforcement member 1402, the openings of the reinforcement member 1402 and the openings 1460 of the surgical pad 1450 can be aligned to facilitate threading therethrough of respective tethers, such as respective pairs of tether tails.

In some instances, the reinforcement member 1402 and surgical pad 1450 can have the same shape. For example, the reinforcement member 1402 and surgical pad 1450 can each comprise a rectangular shape. In some instances, the reinforcement member 1402 and surgical pad 1450 can have the same size, such as having the same footprint. For example, a lateral dimension and a longitudinal dimension of each of the reinforcement member 1402 and surgical pad 1450 can be the same or similar. An outer edge 1408 of the reinforcement member 1402 can be aligned with an outer edge 1456 of the surgical pad 1450 while the surgical pad 1450 is coupled to the first surface 1404 of the reinforcement member 1402. A lateral dimension for each of the reinforcement member 1402 and the surgical pad 1450 can be a dimension extending between opposing portions of the respective outer edges 1408, 1456. A longitudinal dimension of each of the reinforcement member 1402 and surgical pad 1450 can be a dimension extending between opposing portions of the respective outer edges 1408, 1456 and which is perpendicular or substantially perpendicular to the lateral dimension.

It will be understood that a reinforcement member and surgical pad can each comprise another polygonal shape, such as a square. In some instances, a reinforcement member and surgical pad can each comprise a shape having one or more curves, including a circle or oval. For example, the reinforcement member and surgical pad can have the same or similar diameters.

Figures 14A and 14B show the each of the reinforcement member 1402 and the surgical pad 1450 comprising eight openings 1460 extending therethrough, each defined by edges 1458. The eight openings 1460 of the surgical pad 1450 can be arranged in pairs along a longitudinal portion of the surgical pad 1450. For example, the eight openings 1460 can be arranged in four rows, each row having two of the openings 1460. In some instances, the four rows can be evenly spaced, for example each row can have the same spacing from one or more adjacent rows. The reinforcement member 1402 can have openings at corresponding locations such that openings extend through an entire thickness of the reinforced surgical pad device 1400. For example, the reinforcement member 1402 can comprise eight openings. The eight openings can be arranged in four rows, each row comprising two openings.

In some instances, a reinforced surgical pad device can comprise a uniform material. For example, a reinforced surgical pad device can have one or more shapes as described herein, while comprising a material having sufficient mechanical strength to prevent or reduce deformation, warping and/or withstand the forces of the tethers. A reinforced surgical pad can have one or more configurations as described herein and a uniform material throughout, such as rather than a reinforcement member distinct from a surgical pad, and configured to prevent or reduce caving inward of a heart wall.

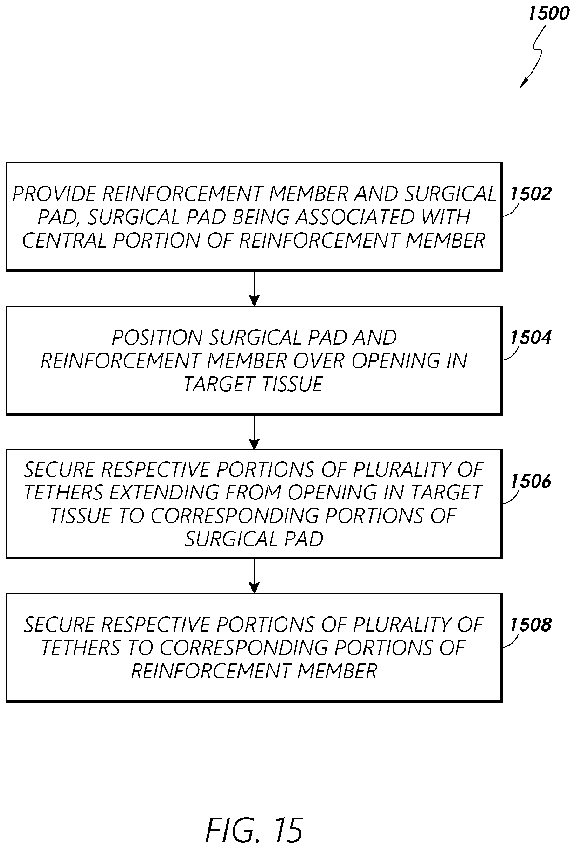

Figure 15 is a flow diagram of an example of a process 1500 for deploying a reinforced surgical pad device. One or more reinforced surgical pad devices as described herein can be used. In block 1502, the process can involve providing a reinforcement member and a surgical pad, the surgical pad being associated with a central portion of the reinforcement member. In some instances, the surgical pad can be coupled to the central portion of the reinforcement member. The reinforcement member can comprise a circumferential portion comprising at least a portion extending laterally beyond an outer edge of the surgical pad.

In block 1504, the process can involve positioning the surgical pad and the reinforcement member over an opening in a target tissue. The surgical pad and/or the reinforcement member can be over and in contact with an externally oriented surface of the target tissue. In block 1506, the process can involve securing respective portions of a plurality of tethers extending from the opening in the target tissue to corresponding portions of the surgical pad. In block 1508, the process can involve securing the respective portions of the plurality of tethers to corresponding portions of the reinforcement member. In some instances, securing the respective portions of the plurality of tethers to corresponding portions of the reinforcement member can comprise securing the respective portions of the plurality of tethers to corresponding tether securement features of the reinforcement member. Alternatively, or in combination, securing the respective portions of the plurality of tethers to corresponding portions of the reinforcement member can comprise wrapping a portion of the plurality of tethers around a portion of the reinforcement member.

Providing the reinforcement member and the surgical pad can comprise providing a reinforcement member comprising a central portion defining an opening extending therethrough, and providing the surgical pad associated with the opening of the reinforcement member. In some instances, providing the surgical pad associated with the opening of the reinforcement member comprises providing the surgical pad disposed within the opening of the reinforcement member.

In some instances, positioning the surgical pad and the reinforcement member over the opening in the target tissue can comprise aligning the surgical pad and the opening of the reinforcement member with the opening in the target tissue. Aligning the surgical pad and the opening of the reinforcement member with the opening in the target tissue can comprise positioning the surgical pad at least partially over the opening in the target tissue. The reinforcement member can be positioned such that the opening in the reinforcement member surrounds the opening in the target tissue. In some instances, the surgical pad can have a footprint larger than that of the opening in the reinforcement member. Positioning the surgical pad and the reinforcement member over the opening in the target tissue can comprise positioning at least a portion of the surgical pad between the reinforcement member and the target tissue. For example, the surgical pad can be aligned with the opening of the reinforcement member and an outer edge portion of the surgical pad can be between the target tissue and the reinforcement member. Securing respective portions of the plurality of tethers to corresponding portions of the surgical pad can comprise advancing the respective portions of the plurality of tethers through the surgical pad. For example, each of the plurality of tethers can be advanced from a first surface of the surgical pad configured to be oriented toward the target tissue through the surgical pad to a second surface of the surgical pad. In some instances, a knot can be formed over the second surface with a respective tether to secure the tether to the surgical pad.

In some instances, providing the reinforcement member and the surgical pad can comprise providing the surgical pad positioned between the target tissue and the reinforcement member. For example, positioning the surgical pad and the reinforcement member over the opening in the target tissue can comprise aligning the surgical pad and the central portion of the reinforcement member with the opening in the target tissue. Positioning the surgical pad and the reinforcement member over the opening in the target tissue can comprise positioning at least a portion of the surgical pad between the central portion of the reinforcement member and the target tissue. Securing respective portions of the plurality of tethers to corresponding portions of the surgical pad can comprise advancing respective portions of the plurality of tethers through the central portions of the reinforcement member and the surgical pad.

The surgical pad and the reinforcement member can be positioned over heart wall tissue. For example, positioning the surgical pad and the reinforcement member over an opening in a target tissue can comprise positioning the surgical pad and the reinforcement member over an externally oriented surface of the heart wall tissue.

## Claims

1. A reinforced surgical pad device (500; 600; 900; 1200), comprising:
a surgical pad (502; 650; 902; 1202) configured to be positioned over an opening (50) in a target tissue (17); and
a reinforcement member (400; 602; 800; 1100) configured to be associated with the
surgical pad (502; 650; 902; 1202), the reinforcement member (400; 602; 800; 1100) comprising:
a central portion (402; 604; 802; 908; 1102) being configured to be associated with the surgical pad (502; 650; 902; 1202), and
a circumferential portion (406; 608; 806; 1106) around the central portion (402; 604; 802; 908; 1102), at least a portion of the circumferential portion (406; 608; 806; 1106) being configured to extend laterally beyond an outer edge of the surgical pad (502; 650; 902; 1202),
wherein the central portion (402; 604; 802; 908; 1102) of the reinforcement member (400; 602; 800; 1100) defines an opening (404; 606; 804; 1104) extending therethrough, the opening (404; 606; 804; 1104) of the reinforcement member (400; 602; 800; 1100) being configured to be aligned with the surgical pad (502; 650; 902; 1202) and the opening (404; 606; 804; 1104) in the target tissue (17), and
wherein the surgical pad (502; 650; 902; 1202) is configured to be disposed within the opening (404; 606; 804; 1104) of the reinforcement member (400; 602; 800; 1100).

2. The device (500; 600; 900; 1200) of claim 1, wherein the circumferential portion (406; 608; 806; 1106) of the reinforcement member (400; 602; 800; 1100) comprises a plurality of elongate portions (612; 808; 1110) extending radially from the central portion (402; 604; 802; 908; 1102) of the reinforcement member (400; 602; 800; 1100).

3. The device (500; 600; 900; 1200) of claim 2, wherein the central portion (402; 604; 802; 908; 1102) comprises a ring-shaped portion.

4. The device (500; 600; 900; 1200) of claim 3, wherein the plurality of elongate portions (612; 808; 1110) extends radially from the ring-shaped portion.

5. The device (500; 600; 900; 1200) of any one of claims 2 to 4, wherein the circumferential portion (406; 608; 806; 1106) comprises one of: four elongate portions (612; 808; 1110) evenly spaced around the central portion (402; 604; 802; 908; 1102), six elongate portions (612; 808; 1110) evenly spaced around the central portion (402; 604; 802; 908; 1102), or eight elongate portions (612; 808; 1110) evenly spaced around the central portion (402; 604; 802; 908; 1102).

6. The device (500; 600; 900; 1200) of any one of claims 2 to 5, wherein each of the plurality of elongate portions (612; 808; 1110) comprises a uniform width along a longitudinal axis.

7. The device (500; 600; 900; 1200) of any one of claims 2 to 5, wherein each of the plurality of elongate portions (612; 808; 1110) comprises a first end portion oriented toward the central portion (402; 604; 802; 908; 1102) and a second end portion oriented away from the central portion (402; 604; 802; 908; 1102), the first end portion having a wider width than the second end portion.

8. The device (500; 600; 900; 1200) of any one of claims 2 to 7, wherein each of the plurality of elongate portions (612; 808; 1110) comprise a uniform thickness along both a longitudinal axis and a lateral axis.

9. The device (500; 600; 900; 1200) of any one of claims 2 to 7, wherein each of the plurality of elongate portions (612; 808; 1110) comprise a longitudinal portion (626) along a longitudinal dimension having a thickness thicker than adjacent portions along the longitudinal dimension.

10. The device (500; 600; 900; 1200) of any one of claims 2 to 9, wherein the reinforcement member (400; 602; 800; 1100) comprises a tether securement feature associated with each of the plurality of elongate portions (612; 808; 1110).

11. The device (500; 600; 900; 1200) of claim 10, wherein each of the plurality of elongate portions (612; 808; 1110) comprise a first end portion oriented toward the central portion (402; 604; 802; 908; 1102) and a second end portion oriented away from the central portion (402; 604; 802; 908; 1102), the tether securement feature being associated with the second end portion of each of the plurality of elongate portions (612; 808; 1110).

12. The device (500; 600; 900; 1200) of claim 2, wherein the circumferential portion (406; 608; 806; 1106) comprises an outer circumferential edge portion (610; 1108), the plurality of elongate portions (612; 808; 1110) extending between the central portion (402; 604; 802; 908; 1102) and the outer circumferential edge portion (610; 1108).

13. The device (500; 600; 900; 1200) of claim 12, wherein the outer circumferential edge portion (610; 1108) comprises an inner edge and an outer edge, the outer edge comprising a square shape and the inner edge comprising a plurality of segments of a circle, each segment of the circle extending between respective adjacent elongate portions (612; 808; 1110), or the outer circumferential edge portion (610; 1108) comprises an inner edge and an outer edge, the outer edge comprising a circular shape and the inner edge comprising a plurality of segments of a circle, each segment of the circle extending between respective adjacent elongate portions (612; 808; 1110).

14. The device (500; 600; 900; 1200) of claim 12 or 13, wherein the reinforcement member (400; 602; 800; 1100) comprises a plurality of tether securement features, the plurality of tether securement features being associated with the outer circumferential edge portion (610; 1108).

15. The device (500; 600; 900; 1200) of claim 14, wherein each of the plurality of elongate portions (612; 808; 1110) comprise a first end portion oriented toward the central portion (402; 604; 802; 908; 1102) and a second end portion oriented toward the outer circumferential edge portion (610; 1108), each of the plurality of tether securement features being disposed between a second end portion of a respective elongate portion and an outer edge of the outer circumferential edge portion (610; 1108).

## Patentansprüche

1. Vorrichtung mit verstärkter chirurgischer Auflage (500; 600; 900; 1200), umfassend:
eine chirurgische Auflage (502; 650; 902; 1202), die dazu konfiguriert ist, über einer Öffnung (50) in einem Zielgewebe (17) positioniert zu werden; und
ein Verstärkungselement (400; 602; 800; 1100), das dazu konfiguriert ist, mit der chirurgischen Auflage (502; 650; 902; 1202) kombiniert zu werden, wobei das Verstärkungselement (400; 602; 800; 1100) umfasst:
einen zentralen Abschnitt (402; 604; 802; 908; 1102), der dazu konfiguriert ist, mit der chirurgischen Auflage (502; 650; 902; 1202) kombiniert zu werden, und
einen umlaufenden Abschnitt (406; 608; 806; 1106) um den zentralen Abschnitt (402; 604; 802; 908; 1102), wobei wenigstens ein Abschnitt des umlaufenden Abschnitts (406; 608; 806; 1106) dazu konfiguriert ist, sich seitlich über einen äußeren Rand der chirurgischen Auflage (502; 650; 902; 1202) hinaus zu erstrecken,
wobei der zentrale Abschnitt (402; 604; 802; 908; 1102) des Verstärkungselements (400; 602; 800; 1100) eine Öffnung (404; 606; 804; 1104) definiert, die sich durch ihn erstreckt, wobei die Öffnung (404; 606; 804; 1104) des Verstärkungselements (400; 602; 800; 1100) dazu konfiguriert ist, auf die chirurgische Auflage (502; 650; 902; 1202) und die Öffnung (404; 606; 804; 1104) im Zielgewebe (17) ausgerichtet zu werden, und
wobei die chirurgische Auflage (502; 650; 902; 1202) dazu konfiguriert ist, innerhalb der Öffnung (404; 606; 804; 1104) des Verstärkungselements (400; 602; 800; 1100) angeordnet zu werden.

2. Vorrichtung (500; 600; 900; 1200) nach Anspruch 1, wobei der umlaufende Abschnitt (406; 608; 806; 1106) des Verstärkungselements (400; 602; 800; 1100) mehrere langgestreckte Abschnitte (612; 808; 1110) umfasst, die sich von dem zentralen Abschnitt (402; 604; 802; 908; 1102) des Verstärkungselements (400; 602; 800; 1100) aus radial erstrecken.

3. Vorrichtung (500; 600; 900; 1200) nach Anspruch 2, wobei der zentrale Abschnitt (402; 604; 802; 908; 1102) einen ringförmigen Abschnitt umfasst.

4. Vorrichtung (500; 600; 900; 1200) nach Anspruch 3, wobei sich die mehreren langgestreckten Abschnitte (612; 808; 1110) von dem ringförmigen Abschnitt aus radial erstrecken.

5. Vorrichtung (500; 600; 900; 1200) nach einem der Ansprüche 2 bis 4, wobei der umlaufende Abschnitt (406; 608; 806; 1106) eines umfasst von: vier langgestreckte Abschnitte (612; 808; 1110), die um den zentralen Abschnitt (402; 604; 802; 908; 1102) gleichmäßig beabstandet angeordnet sind, sechs langgestreckte Abschnitte (612; 808; 1110), die um den zentralen Abschnitt (402; 604; 802; 908; 1102) gleichmäßig beabstandet angeordnet sind, oder acht langgestreckte Abschnitte (612; 808; 1110), die um den zentralen Abschnitt (402; 604; 802; 908; 1102) gleichmäßig beabstandet angeordnet sind.

6. Vorrichtung (500; 600; 900; 1200) nach einem der Ansprüche 2 bis 5, wobei jeder der mehreren langgestreckten Abschnitte (612; 808; 1110) eine gleichmäßige Breite entlang einer Längsachse aufweist.

7. Vorrichtung (500; 600; 900; 1200) nach einem der Ansprüche 2 bis 5, wobei jeder der mehreren langgestreckten Abschnitte (612; 808; 1110) einen ersten Endabschnitt, der in Richtung zu dem zentralen Abschnitt (402; 604; 802; 908; 1102) gerichtet ist, und einen zweiten Endabschnitt, der von dem zentralen Abschnitt (402; 604; 802; 908; 1102) weg gerichtet ist, aufweist, wobei der erste Endabschnitt eine größere Breite als der zweite Endabschnitt aufweist.

8. Vorrichtung (500; 600; 900; 1200) nach einem der Ansprüche 2 bis 7, wobei jeder der mehreren langgestreckten Abschnitte (612; 808; 1110) eine gleichmäßige Dicke entlang sowohl einer Längsachse als auch einer Querachse aufweist.

9. Vorrichtung (500; 600; 900; 1200) nach einem der Ansprüche 2 bis 7, wobei jeder der mehreren langgestreckten Abschnitte (612; 808; 1110) einen longitudinalen Abschnitt (626) entlang einer Längsabmessung umfasst, der eine größere Dicke aufweist als benachbarte Abschnitte entlang der Längsabmessung.

10. Vorrichtung (500; 600; 900; 1200) nach einem der Ansprüche 2 bis 9, wobei das Verstärkungselement (400; 602; 800; 1100) ein Halteleinen-Befestigungsmerkmal umfasst, das mit jedem der mehreren langgestreckten Abschnitte (612; 808; 1110) kombiniert ist.

11. Vorrichtung (500; 600; 900; 1200) nach Anspruch 10, wobei jeder der mehreren langgestreckten Abschnitte (612; 808; 1110) einen ersten Endabschnitt, der in Richtung zu dem zentralen Abschnitt (402; 604; 802; 908; 1102) gerichtet ist, und einen zweiten Endabschnitt, der von dem zentralen Abschnitt (402; 604; 802; 908; 1102) weg gerichtet ist, umfasst, wobei das Halteleinen-Befestigungsmerkmal mit dem zweiten Endabschnitt jedes der mehreren langgestreckten Abschnitte (612; 808; 1110) kombiniert ist.

12. Vorrichtung (500; 600; 900; 1200) nach Anspruch 2, wobei der umlaufende Abschnitt (406; 608; 806; 1106) einen äußeren umlaufenden Randabschnitt (610; 1108) umfasst, wobei sich die mehreren langgestreckten Abschnitte (612; 808; 1110) zwischen dem zentralen Abschnitt (402; 604; 802; 908; 1102) und dem äußeren umlaufenden Randabschnitt (610; 1108) erstrecken.

13. Vorrichtung (500; 600; 900; 1200) nach Anspruch 12, wobei der äußere umlaufende Randabschnitt (610; 1108) einen inneren Rand und einen äußeren Rand umfasst, wobei der äußere Rand eine quadratische Form aufweist und der innere Rand mehrere Segmente eines Kreises aufweist, wobei sich jedes Segment des Kreises zwischen jeweils benachbarten langgestreckten Abschnitten (612; 808; 1110) erstreckt, oder der äußere umlaufende Randabschnitt (610; 1108) einen inneren Rand und einen äußeren Rand umfasst, wobei der äußere Rand eine Kreisform aufweist und der innere Rand mehrere Segmente eines Kreises aufweist, wobei sich jedes Segment des Kreises zwischen jeweils benachbarten langgestreckten Abschnitten (612; 808; 1110) erstreckt.

14. Vorrichtung (500; 600; 900; 1200) nach Anspruch 12 oder 13, wobei das Verstärkungselement (400; 602; 800; 1100) mehrere Halteleinen-Befestigungsmerkmale umfasst, wobei die mehreren Halteleinen-Befestigungsmerkmale mit dem äußeren umlaufenden Randabschnitt (610; 1108) verbunden sind.

15. Vorrichtung (500; 600; 900; 1200) nach Anspruch 14, wobei jeder der mehreren langgestreckten Abschnitte (612; 808; 1110) einen ersten Endabschnitt, der in Richtung zu dem zentralen Abschnitt (402; 604; 802; 908; 1102) gerichtet ist, und einen zweiten Endabschnitt, der in Richtung zu dem äußeren umlaufenden Randabschnitt (610; 1108) gerichtet ist, umfasst, wobei jedes der mehreren Halteleinen-Befestigungsmerkmale zwischen einem zweiten Endabschnitt eines jeweiligen langgestreckten Abschnitts und einem äußeren Rand des äußeren umlaufenden Randabschnitts (610; 1108) angeordnet ist.

## Revendications

1. Dispositif (500 ; 600 ; 900 ; 1200) de tampon chirurgical renforcé, comprenant :
un tampon chirurgical (502 ; 650 ; 902 ; 1202) conçu pour être positionné sur une ouverture (50) dans un tissu cible (17) ; et
un élément de renforcement (400 ; 602 ; 800 ; 1100) conçu pour être associé au tampon chirurgical (502 ; 650 ; 902 ; 1202), l'élément de renforcement (400 ; 602 ; 800 ; 1100) comprenant :
une partie centrale (402 ; 604 ; 802 ; 908 ; 1102) conçue pour être associée au tampon chirurgical (502 ; 650 ; 902 ; 1202) et
une partie circonférentielle (406 ; 608 ; 806 ; 1106) autour de la partie centrale (402 ; 604 ; 802; 908 ; 1102), au moins une partie de la partie circonférentielle (406 ; 608 ; 806 ; 1106) étant conçue pour s'étendre latéralement au-delà d'un bord externe du tampon chirurgical (502 ; 650 ; 902 ; 1202),
la partie centrale (402 ; 604 ; 802 ; 908 ; 1102) de l'élément de renforcement (400 ; 602 ; 800 ; 1100) définissant une ouverture (404 ; 606 ; 804 ; 1104) s'étendant à travers celle-ci, l'ouverture (404 ; 606 ; 804 ; 1104) de l'élément de renforcement (400 ; 602 ; 800 ; 1100) étant conçue pour être alignée sur le tampon chirurgical (502 ; 650 ; 902 ; 1202) et sur l'ouverture (404 ; 606 ; 804 ; 1104) dans le tissu cible (17),
le dispositif de tampon chirurgical renforcé étant **caractérisé en ce que** le tampon chirurgical (502 ; 650 ; 902 ; 1202) est conçu pour être disposé à l'intérieur de l'ouverture (404 ; 606 ; 804 ; 1104) de l'élément de renforcement (400 ; 602 ; 800 ; 1100).

2. Dispositif (500 ; 600 ; 900 ; 1200) selon la revendication 1, la partie circonférentielle (406 ; 608 ; 806 ; 1106) de l'élément de renforcement (400 ; 602 ; 800 ; 1100) comprenant une pluralité de parties allongées (612 ; 808 ; 1110) s'étendant radialement à partir de la partie centrale (402 ; 604 ; 802 ; 908 ; 1102) de l'élément de renforcement (400 ; 602 ; 800 ; 1100).

3. Dispositif (500 ; 600 ; 900 ; 1200) selon la revendication 2, la partie centrale (402 ; 604 ; 802 ; 908 ; 1102) comprenant une partie en forme d'anneau.

4. Dispositif (500 ; 600 ; 900 ; 1200) selon la revendication 3, la pluralité de parties allongées (612 ; 808 ; 1110) s'étendant radialement à partir de la partie en forme d'anneau.

5. Dispositif (500 ; 600 ; 900 ; 1200) selon l'une quelconque des revendications 2 à 4, la partie circonférentielle (406 ; 608 ; 806 ; 1106) présentant une caractéristique parmi : quatre parties allongées (612 ; 808 ; 1110) espacées régulièrement autour de la partie centrale (402 ; 604 ; 802 ; 908 ; 1102), six parties allongées (612 ; 808 ; 1110) espacées régulièrement autour de la partie centrale (402 ; 604 ; 802 ; 908 ; 1102) ou huit parties allongées (612 ; 808 ; 1110) espacées régulièrement autour de la partie centrale (402 ; 604 ; 802 ; 908 ; 1102).

6. Dispositif (500 ; 600 ; 900 ; 1200) selon l'une quelconque des revendications 2 à 5, chaque partie parmi la pluralité de parties allongées (612 ; 808 ; 1110) présentant une largeur uniforme le long d'un axe longitudinal.

7. Dispositif (500 ; 600 ; 900 ; 1200) selon l'une quelconque des revendications 2 à 5, chaque partie parmi la pluralité de parties allongées (612 ; 808 ; 1110) comprenant une première partie d'extrémité orientée vers la partie centrale (402 ; 604 ; 802 ; 908 ; 1102) et une seconde partie d'extrémité orientée à l'opposé de la partie centrale (402 ; 604 ; 802 ; 908 ; 1102), la première partie d'extrémité présentant une largeur plus large que celle de la seconde partie d'extrémité.

8. Dispositif (500 ; 600 ; 900 ; 1200) selon l'une quelconque des revendications 2 à 7, chaque partie parmi la pluralité de parties allongées (612 ; 808 ; 1110) présentant une épaisseur uniforme le long à la fois d'un axe longitudinal et d'un axe latéral.

9. Dispositif (500 ; 600 ; 900 ; 1200) selon l'une quelconque des revendications 2 à 7, chaque partie parmi la pluralité de parties allongées (612 ; 808 ; 1110) comprenant une partie longitudinale (626) le long d'une dimension longitudinale présentant une épaisseur plus épaisse que celle de parties adjacentes le long de la dimension longitudinale.

10. Dispositif (500 ; 600 ; 900 ; 1200) selon l'une quelconque des revendications 2 à 9, l'élément de renforcement (400 ; 602 ; 800 ; 1100) comprenant une caractéristique de fixation d'attache associée à chaque partie parmi la pluralité de parties allongées (612 ; 808 ; 1110).

11. Dispositif (500 ; 600 ; 900 ; 1200) selon la revendication 10, chaque partie parmi la pluralité de parties allongées (612 ; 808 ; 1110) comprenant une première partie d'extrémité orientée vers la partie centrale (402 ; 604 ; 802 ; 908 ; 1102) et une seconde partie d'extrémité orientée à l'opposé de la partie centrale (402 ; 604 ; 802 ; 908 ; 1102), l'élément de fixation d'attache étant associé à la seconde partie d'extrémité de chaque partie parmi la pluralité de parties allongées (612 ; 808 ; 1110).

12. Dispositif (500 ; 600 ; 900 ; 1200) selon la revendication 2, la partie circonférentielle (406 ; 608 ; 806 ; 1106) comprenant une partie de bord circonférentiel externe (610 ; 1108), la pluralité de parties allongées (612 ; 808 ; 1110) s'étendant entre la partie centrale (402 ; 604 ; 802 ; 908 ; 1102) et la partie de bord circonférentiel externe (610 ; 1108).

13. Dispositif (500 ; 600 ; 900 ; 1200) selon la revendication 12, la partie de bord circonférentiel externe (610 ; 1108) comprenant un bord interne et un bord externe, le bord externe présentant une forme carrée et le bord interne comprenant une pluralité de segments d'un cercle, chaque segment du cercle s'étendant entre des parties allongées adjacentes (612 ; 808 ; 1110) respectives ou la partie de bord circonférentiel externe (610 ; 1108) comprenant un bord interne et un bord externe, le bord externe présentant une forme circulaire et le bord interne comprenant une pluralité de segments d'un cercle, chaque segment du cercle s'étendant entre des parties allongées adjacentes (612 ; 808 ; 1110) respectives.

14. Dispositif (500 ; 600 ; 900 ; 1200) selon la revendication 12 ou 13, l'élément de renforcement (400 ; 602 ; 800 ; 1100) comprenant une pluralité de caractéristiques de fixation d'attache, la pluralité de caractéristiques de fixation d'attache étant associée à la partie de bord circonférentiel externe (610 ; 1108).

15. Dispositif (500 ; 600 ; 900 ; 1200) selon la revendication 14, chaque partie parmi la pluralité de parties allongées (612 ; 808 ; 1110) comprenant une première partie d'extrémité orientée vers la partie centrale (402 ; 604 ; 802 ; 908 ; 1102) et une seconde partie d'extrémité orientée vers la partie de bord circonférentiel externe (610 ; 1108), chaque caractéristique parmi la pluralité de caractéristiques de fixation d'attache étant disposée entre une seconde partie d'extrémité d'une partie allongée respective et un bord externe de la partie de bord circonférentiel externe (610 ; 1108).
